# EUROPEAN PATENT APPLICATION

(11) **EP 4 574 839 A1**
(43) Date of publication of application: **25.06.2025**
(21) Application number: 23219851.5
(22) Date of filing: 22.12.2023
(51) Int. Cl.: C07K 16/00

(54) **CO-DELIVERY SYSTEM**

(71) Applicant: BioNTech SE, 55131 Mainz (DE)
(72) Inventor: BOGEN, Jan Patrick, 55131 Mainz (DE); WÜSTEHUBE-LAUSCH, Joycelyn, 55131 Mainz (DE); SCHMOLDT, Hans-Ulrich, 55131 Mainz (DE); SAHIN, Ugur, 55131 Mainz (DE)
(74) Representative: D Young & Co LLP

(57) **Abstract**

The present invention relates to a co-delivery system comprising one or more nucleic acids encoding a first antibody and a second antibody. In particular, the co-delivery system encodes a first antibody and a second antibody each comprising two antibody chains, and wherein the two antibody chains of each antibody are covalently linked by at least one inter-chain disulphide bond located at a different position in each of the two antibodies. The invention also relates to a vector comprising the co-delivery system, a nucleic acid particle comprising the co-delivery system and a composition comprising the co-delivery system as well as uses of the co-delivery system in a method of therapy and/or a diagnostic method.

## Description

### FIELD OF THE INVENTION

The present invention relates to a co-delivery system comprising one or more nucleic acids encoding a first antibody and a second antibody. In particular, the co-delivery system encodes a first antibody and a second antibody each comprising two antibody chains, and wherein the two antibody chains of each antibody are covalently linked by at least one inter-chain disulphide bond located at a different position in each of the two antibodies. The invention also relates to a vector comprising the co-delivery system, a nucleic acid particle comprising the co-delivery system and a composition comprising the co-delivery system as well as uses of the co-delivery system in a method of therapy and/or a diagnostic method.

### BACKGROUND OF THE INVENTION

Antibody-based therapeutics have grown rapidly within the field of immune-oncologic treatments. By blocking a receptor on a cancerous target cell (inhibition) or mediating cytotoxic effector functions (CDC/ADCC/ADCP), monoclonal antibodies (mAb) can mediate different effects on a target cell. However, most antibodies rely on a single mode of action (MOA), from which a cell can escape by downregulating the expression of the targeted antigen on its cell surface, by suppressing the immunological capabilities of immune cells in the tumour microenvironment or switching to another growth-inducing pathway. All these mechanisms lead to a loss of efficacy of the therapeutic mAb, limiting its effectiveness. To circumvent these events, antibody combinations are becoming increasingly popular.

Possible combination rationales include the blockage of two different cancer cell pathways which both mediate tumour progression, like blockage of the EGFR and VEGF pathway (Bevacizumab and Panitumumab). Other rationales are based on blocking different immune checkpoints like PD-1 and CTLA-4, in order to circumvent T cell suppression (Ipilimumab and Nivolumab). Clinical studies of combinations of antibodies targeting the same antigen but at different epitopes are also reported, such as the anti-Her2 antibodies Trastuzumab and Pertuzumab. This combination facilitates a stronger clustering of HER2 and might support the clearance of it from the cell surface as well as elevating ADCC activities. Several known antibody combinations are under clinical investigation, and some have already gained regulatory approval.

The benefit of these combinations lies in the fact that mAbs often have only a limited overlapping toxicity and few to no pharmacokinetic interactions with one another.

However, producing two separate antibodies in an expensive and highly regulated GMP environment, leads to increased costs of a combination therapy. Here, the inventors seek to provide ways to simplify the production of combination antibody products.

It was previously shown that mRNA encoding an antibody can be delivered to the liver of animals and humans via lipid nanoparticles (LNP). In the liver, the mRNA is translated into the antibody, which is secreted into the blood stream, becoming systemically available.

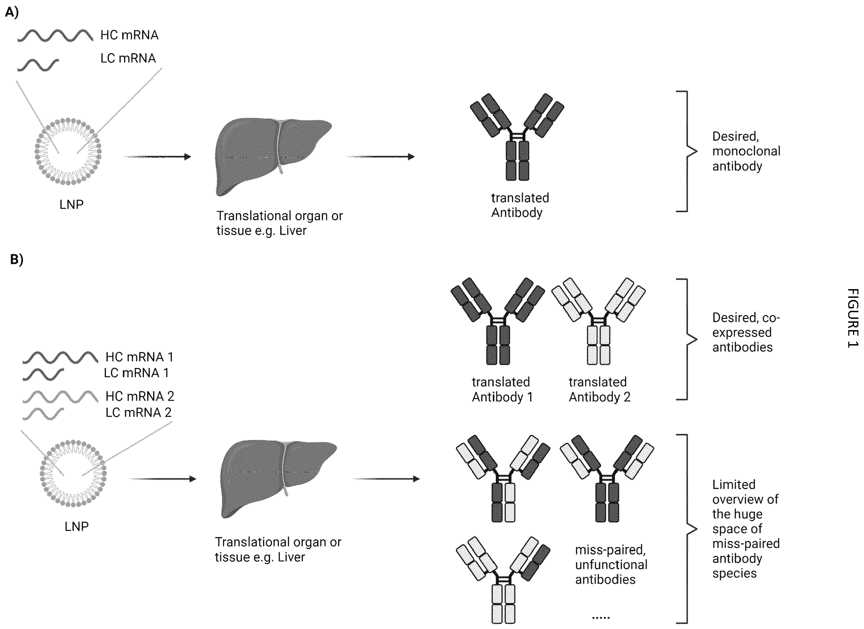

Traditionally, antibodies consist of multiple polypeptide chains. For example, an IgG1 antibody comprises one heavy chain (HC) and one light chain (LC) sequence which are aligned in a 2:2 tetrameric manner and form the active molecule (Figure 1A). The co-translation of multiple antibodies, for example within one particle or LNP, can lead to chain-scrambled and non-active antibodies upon translation. For example, the heavy chain (HC) of one IgG1 antibody may interact with the heavy chain of a second IgG1 antibody for HC:HC paring or the light chain (LC) of one antibody may bind to the heavy chain of a second antibody (Figure 1B). These scrambled antibodies are not functional, or at least are not fully functional. Further, this process of chain scrambling can lead to a massive heterogenicity within the produced antibodies.

The present invention addresses these and other limitations in the art.

### SUMMARY OF THE INVENTION

The present invention is based, at least in part, on the inventor's development of a co-delivery system that has particularly advantageous and surprising properties. The present inventors have developed a co-delivery system which facilitates a combination therapy of nucleotide encoded (e.g. mRNA encoded) antibodies. By utilising antibodies which are differentially stabilised - for example, with one antibody being stabilised through the hinge region and another being stabilised outside the hinge region - destabilisation of mispaired chains and stabilisation (e.g. covalent stabilisation) of correctly paired antibodies is achieved. This is advantageous since it facilitates the formation of correctly paired antibody chains, including of multispecific antibodies. In other words, such a co-delivery system avoids mispairing and allows multiple antibodies to be produced with increased integrity.

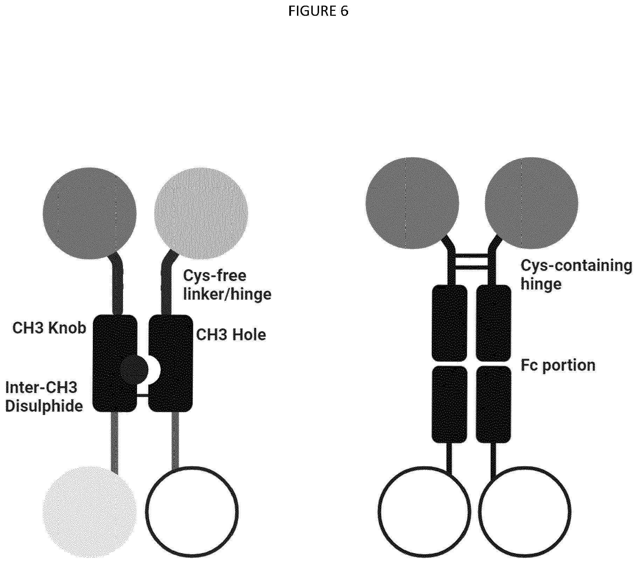

The present inventors have surprisingly shown that this co-delivery system provides a simplified way in which to produce a combination antibody product, and also ensure the integrity of each antibody within the combination antibody product, by ensuring that the chains of each antibody pair appropriately. The antibodies encoded by the co-delivery system may include IgG-like antibodies and VHH-CH3 fusions, but this technology also allows for the usage of other fusion partners as well (see Figure 6).

Here the inventors have demonstrated a novel technology to co-express multiple antibody-derived fragments within a single cell resulting in exclusive formation of the desired products. In particular, the inventors have demonstrated the feasibility of the formulation of multiple RNAs, encoding different antibody-derived polypeptide chains, into a single particle (such as an LNP), and the subsequent translation into fully functional and correctly paired multi-specific antibodies.

Accordingly, in a first aspect the invention provides a co-delivery system comprising one or more nucleic acid molecules encoding a first antibody and a second antibody,
wherein each of the first antibody and the second antibody comprises at least a first polypeptide chain and a second polypeptide chain covalently linked by at least one inter-chain disulphide bond, and
wherein all of the inter-chain disulphide bonds between the first polypeptide chain and the second polypeptide chain of the first antibody are at different positions to the inter-chain disulphide bonds between the first polypeptide chain and the second polypeptide chain of the second antibody.

In some embodiments, all of the inter-chain disulphide bonds between the first polypeptide chain and the second polypeptide chain of the first antibody are within the hinge region.

In some embodiments, all of the inter-chain disulphide bonds between the first polypeptide chain and the second polypeptide chain of the second antibody are outside the hinge region.

Accordingly, in a further aspect the invention provides a co-delivery system comprising one or more nucleic acid molecules encoding a first antibody and a second antibody,
wherein each of the first antibody and the second antibody comprises at least a first polypeptide chain and a second polypeptide chain,
wherein the first polypeptide chain and the second polypeptide chain of the first antibody are covalently linked by at least one inter-chain disulphide bond within the hinge region,
wherein the first polypeptide chain and the second polypeptide chain of the second antibody are covalently linked by at least one inter-chain disulphide bond, and
wherein all of the inter-chain disulphide bonds between the first polypeptide chain and the second polypeptide chain of the second antibody are outside the hinge region.

In some embodiments, each nucleic acid molecule encodes at least one polypeptide chain of an antibody.

In some embodiments, the nucleic acid molecule(s) is DNA and/or RNA.

In some embodiments, the first polypeptide chain of the first antibody comprises at least one binding moiety or at least one cognate of a binding moiety at the N- and/or C-terminus.

In some embodiments, the first polypeptide chain of the second antibody comprises at least one binding moiety or at least one cognate of a binding moiety at the N- and/or C-terminus.

In some embodiments, the second polypeptide chain of the first antibody comprises at least one binding moiety or at least one cognate of a binding moiety at the N- and/or C-terminus.

In some embodiments, the second polypeptide chain of the second antibody comprises at least one binding moiety or at least one cognate of a binding moiety at the N- and/or C-terminus.

In some embodiments, the binding moiety is selected from the list consisting of a single-chain variable fragment (scFv); a Fab; a Fab'; a F(ab)'2; an Fv; a single domain antibody (sdAb); a VHH; a single chain variable domain; a designed ankyrin repeat protein (DARPin); and an aptamer.

In some embodiments, the first and second antibodies are independently selected from the group consisting of a human antibody, a humanized antibody, a chimeric antibody, a multispecific antibody, a monoclonal antibody, a polyclonal antibody, or any combination thereof.

In some embodiments, the first and/or second antibody is a multispecific antibody.

In some embodiments, each of the first and second antibodies is a multispecific antibody.

In some embodiments, the one or more nucleic acid molecules further encodes one or more additional antibodies, preferably one, two, three, four or five additional antibodies.

In some embodiments, the first antibody comprises an Fc region.

In some embodiments, the first antibody is an IgG antibody.

In some embodiments, the first polypeptide chain and the second polypeptide chain of the second antibody each further comprises one or more constant heavy 3 (CH3) domains.

In some embodiments, the second antibody is a VHH-CH3 fusion.

In a further aspect the invention provides a co-delivery system comprising one or more nucleic acid molecules encoding a first antibody and a second antibody,
wherein each of the first antibody and the second antibody comprises at least a first polypeptide chain and a second polypeptide chain covalently linked by at least one inter-chain disulphide bond,
wherein all of the inter-chain disulphide bonds of the first antibody are at different positions to the inter-chain disulphide bonds of the second antibody,
wherein the first antibody is an IgG antibody, and
wherein the second antibody is a VHH-CH3 fusion.

In some embodiments, the first polypeptide chain and the second polypeptide chain of the second antibody each comprises a single CH3 domain.

In some embodiments, one or more of the CH3 domains of the first polypeptide chain and/or one or more of the CH3 domains of the second polypeptide chain of the second antibody comprise a modification that enhances the formation of the antibody.

In some embodiments, one or more of the CH3 domains of the first polypeptide chain and/or one or more of the CH3 domains of the second polypeptide chain of the second antibody comprise a modification that enhances the affinity between the first and second polypeptide chains.

In some embodiments, one or more of the CH3 domains of the first polypeptide chain and/or one or more of the CH3 domains of the second polypeptide chain of the second antibody comprise a modification that reduces steric hindrance to the formation of the antibody.

In some embodiments, one or more of the CH3 domains of the first polypeptide chain and one or more of the CH3 domains of the second polypeptide chain of the second antibody are associated via a 'knobs-into-holes' modification.

In some embodiments, the second antibody is a heterodimer.

In some embodiments, at least one inter-chain disulphide bond is formed between a CH3 domain of the first polypeptide chain and a CH3 domain of the second polypeptide chain of the second antibody.

In some embodiments, at least one inter-chain disulphide bond of the second antibody is an asymmetric disulphide bond.

In some embodiments, one or more of the CH3 domains of the first polypeptide chain and one or more of the CH3 domains of the second polypeptide chain of the second antibody comprise a modification to introduce a cysteine residue to promote the formation of a disulphide bond between the CH3 domains of the first and second polypeptide chains.

In some embodiments, the modification(s) to introduce a cysteine residue to promote the formation of a disulphide bond between the CH3 domains of the first and second polypeptide chains are configured to provide an asymmetric disulphide bond.

In some embodiments, the first polypeptide chain of the second antibody comprises one or more constant immunoglobulin domains and one or more variable immunoglobulin domains N-terminal of the constant immunoglobulin domains, wherein all of the variable immunoglobulin domains N-terminal of the constant immunoglobulin domains of the first polypeptide chain are single domain variable heavy immunoglobulin (VHH) domains, and wherein the second polypeptide chain of the second antibody comprises one or more constant immunoglobulin domains, wherein all of the constant immunoglobulin domains in the first polypeptide chain and the second polypeptide chain are constant heavy 3 (CH3) domains.

In some embodiments, the second polypeptide chain of the second antibody further comprises one or more variable immunoglobulin domains N-terminal of the constant immunoglobulin domains, wherein all of the variable immunoglobulin domains N-terminal of the CH3 domains are single domain variable heavy immunoglobulin (VHH) domains.

In some embodiments, the first polypeptide chain and the second polypeptide chain of the second antibody each comprises a single VHH domain N-terminal of the CH3 domains.

In some embodiments, the first polypeptide chain of the second antibody further comprises at least one binding moiety or at least one cognate of a binding moiety C-terminal of the CH3 domains.

In some embodiments:
a) within the first polypeptide chain of the second antibody the sequence between the CH3 domain(s) and the VHH domain(s) located N-terminal of the CH3 domain(s) consists of a first linking sequence; and
b) within the second polypeptide chain of the second antibody the sequence between the CH3 domain(s) and the VHH domains located N-terminal of the CH3 domains consists of a second linking sequence.

In some embodiments, each of the one or more additional antibodies comprises at least a first polypeptide chain and a second polypeptide chain, wherein the first polypeptide chain and the second polypeptide chain are covalently linked by at least one inter-chain disulphide bond, and wherein all of the inter-chain disulphide bonds between the first polypeptide chain and the second polypeptide chain of the one or more additional antibodies are at different positions to the inter-chain disulphide bonds between the first polypeptide chain and the second polypeptide chain in each of the first antibody and the second antibody.

In some embodiments, all of the inter-chain disulphide bonds between the first polypeptide chain and the second polypeptide chain of the one or more additional antibodies are outside the hinge region.

In some embodiments, the first polypeptide chain and the second polypeptide chain of each of the one or more additional antibodies each further comprises one or more constant heavy 3 (CH3) domains.

In some embodiments, one or more of the CH3 domains of the first polypeptide chain and/or one or more of the CH3 domains of the second polypeptide chain of each of the one or more additional antibodies comprise a modification that enhances the formation of the antibody.

In some embodiments, one or more of the CH3 domains of the first polypeptide chain and/or one or more of the CH3 domains of the second polypeptide chain of the second antibody and of each of the one or more additional antibodies comprise different modifications that enhance the formation of the antibody.

In some embodiments, one or more of the CH3 domains of the first polypeptide chain and/or one or more of the CH3 domains of the second polypeptide chain of the second antibody and of each of the one or more additional antibodies comprise the same modification that enhances the formation of the antibody.

In some embodiments, each of the one or more additional antibodies comprises at least a first polypeptide chain and a second polypeptide chain, wherein the first polypeptide chain and the second polypeptide chain are covalently linked by at least one inter-chain disulphide bond, wherein the first polypeptide chain and the second polypeptide chain of each of the one or more additional antibodies each further comprises one or more constant heavy 3 (CH3) domains, wherein one or more of the CH3 domains of the first polypeptide chain and/or one or more of the CH3 domains of the second polypeptide chain of each of the one or more additional antibodies comprises a modification that enhances the formation of the antibody, and wherein the first polypeptide chain of the second antibody and the first polypeptide chain of each of the one or more additional antibodies is identical.

In some embodiments, the second polypeptide chain of the second antibody is different to the second polypeptide chain of each of the one or more additional antibodies.

In some embodiments, one or more of the CH3 domains of the first polypeptide and one or more of the CH3 domains of the second polypeptide of each of the one or more additional antibodies are associated via a 'knobs-into-holes' modification.

In some embodiments, at least one inter-chain disulphide bond of each of the one or more additional antibodies is an asymmetric disulphide bond.

In some embodiments, the co-delivery system further comprises a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein all of the inter-chain disulphide bonds between the first polypeptide chain and the second polypeptide chain of the third antibody are at different positions to the inter-chain disulphide bonds between the first polypeptide chain and the second polypeptide chain in each of the first antibody and the second antibody, and wherein one or more of the CH3 domains of the first polypeptide and one or more of the CH3 domains of the second polypeptide of the third antibody comprise a modification that promotes electrostatic interactions between the first and second polypeptide chains of the third antibody.

In some embodiments, all of the inter-chain disulphide bonds between the first polypeptide chain and the second polypeptide chain of the third antibody are outside the hinge region.

In some embodiments, the co-delivery system further comprises a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein the first polypeptide chain and the second polypeptide chain of the third antibody each further comprises one or more constant heavy 3 (CH3) domains, wherein one or more of the CH3 domains of the first polypeptide chain and/or one or more of the CH3 domains of the second polypeptide chain of the third antibody comprises a modification that enhances the formation of the antibody, and wherein the first polypeptide chain of the second antibody and the first polypeptide chain of the third antibody is identical.

In some embodiments, the second polypeptide chain of the second antibody is different to the second polypeptide chain of the third antibody.

In some embodiments, the co-delivery system further comprises a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond formed between a CH3 domain of the first polypeptide and a CH3 domain of the second polypeptide, and wherein all of the inter-chain disulphide bonds between the first polypeptide chain and the second polypeptide chain of the third antibody are outside the hinge region.

In some embodiments, at least one inter-chain disulphide bond of the third antibody is an asymmetric disulphide bond.

In some embodiments, when at least one inter-chain disulphide bond of the second antibody is an asymmetric disulphide bond, the asymmetric disulphide bond of the second antibody is different to the asymmetric disulphide bond of the third antibody.

In some embodiments, one or more of the CH3 domains of the first polypeptide chain and one or more of the CH3 domains of the second polypeptide chain of the third antibody are associated via a 'knobs-into-holes' modification.

In a further aspect, the invention provides a vector comprising the co-delivery system of the invention.

In a further aspect, the invention provides a nucleic acid particle comprising the co-delivery system of the invention or the vector of the invention.

In a further aspect, the invention provides a composition comprising the co-delivery system of the invention, the vector of the invention or the nucleic acid particle of the invention.

In a further aspect, the invention provides the co-delivery system of the invention, the vector of the invention, the nucleic acid particle of the invention, or the composition of the invention for use in a method of therapy and/or in a diagnostic method.

### BRIEF DESCRIPTION OF THE FIGURES

**Figure 1****. mRNA-encoded antibody production and chain pairing. A)** mRNA encoding the LC and HC on an IgG1 antibody is translated in the liver to one functional monoclonal, tetrameric antibody. B) Co-transport of four mRNAs, encoding for LC and HC of two antibodies will lead to translation into various antibody species which differ in chain pairing with very limited functionality and huge heterogenicity.
**Figure 2****. Theoretical Chain Pairing: Intra-construct chain mispairing.**
**Figure 3****. Theoretical Chain Pairing: Inter-construct chain mispairing.**
**Figure 4****. Theoretical Chain Pairing: Inter-construct chain pairing.**
**Figure 5****. Co-transfection experiments using multiple hole chains.**
**Figure 6****. Important components of embodiments of the co-delivery system of the invention.** Black structures show the preferred differential stabilisations of correctly paired chains, this includes Knobs-into-Holes-CH3 domains exhibiting an asymmetrical disulphide bridge for one antibody (left hand panel) and a Hinge-disulphide-stabilized Fc portion for the other antibody (right hand panel). Grey circles indicate interchangeability for different protein subunits, for example, but not limited to variable domains including VHHs, Fabs and DARPins. The same colour reflects same specificity, but this does not limit the number of binding moieties.
**Figure 7****. Co-delivery of VHH-CH3 fusions and IgG-like constructs.**
**Figure 8****. Best Ratio for Co-Delivery of VHH-CH3 fusion and IgG-like antibody.** Comparison of Western Blot and highly sensitive Jess Western Blots for the best molar ratio.
**Figure 9****. Co-transfection of two trispecific VHH-CH3 fusions and one bispecific IgG-like antibody.**

### DETAILED DESCRIPTION

Conventional delivery approaches for combination antibody therapies rely on the production of the two antibodies separately in an expensive and highly regulated GMP environment, increasing costs of the combination therapy.

Recently, the delivery of mRNA-encoded antibodies has been developed for single antibody therapies. The present inventors have recognised that, in principle, lipid nanoparticle technology allows for the encapsulation of multiple mRNA-encoded antibodies into one particle. However, this will lead to chain-scrambled and non-active antibodies upon translation as the respective antibody chains can interact with both intra-construct and inter-construct mispairings. Such mispaired (i.e. scrambled) antibodies are not, or at least not fully, functional and would lead to a massive heterogeneity within the produced antibodies.

The present inventors have developed a co-delivery system to co-express multiple antibodies within a single cell resulting in exclusive formation of the desired products. Hence, the inventors provide a simplified way in which to produce a combination antibody product in single cell, thereby providing an efficient and cost effective combination antibody therapy. Moreover, the co-delivery system provided herein utilises differential stabilisations of the correctly paired antibody chains to ensure the integrity of each antibody within the combination antibody product, i.e. avoids mispairing and stabilises correctly formed antibodies, thereby allowing multiple antibodies to be produced with the correct chain pairings.

In particular, the present inventors have engineered the two antibodies for co-delivery to contain disulphide bonds in different positions of the heavy chains of the antibodies. This promotes the formation of antibodies having the correct chain pairings by covalently stabilising the correctly paired antibody products. Moreover, the present inventors have also demonstrated that further engineering of one of the antibodies to contain a Knobs-into-Holes (KiH) modification promotes formation of the correctly paired heterodimer as well as destabilising the intra-construct mispaired antibody products, for example by steric hindrance.

The present inventors have surprisingly demonstrated that the co-delivery system of the invention permits the co-expression of multiple antibodies within a single cell and results in exclusive formation of the desired products. In particular, the inventors have demonstrated the co-expression of at least three antibodies, based upon five different polypeptide chains, within a single cell. The inventors have also verified that the antibodies are fully functional.

### Co-delivery system

The present invention generally relates to a co-delivery system.

Accordingly, in a first aspect the invention provides a co-delivery system comprising one or more nucleic acid molecules encoding a first antibody and a second antibody, wherein each of the first antibody and the second antibody comprises at least a first polypeptide chain and a second polypeptide chain covalently linked by at least one inter-chain disulphide bond, and wherein all of the inter-chain disulphide bonds between the first polypeptide chain and the second polypeptide chain of the first antibody are at different positions to the inter-chain disulphide bonds between the first polypeptide chain and the second polypeptide chain of the second antibody.

In some embodiments, all of the at least one inter-chain disulphide bonds between the first polypeptide chain and the second polypeptide chain of the first antibody is within the hinge region.

In some embodiments, all of the inter-chain disulphide bonds between the first polypeptide chain and the second polypeptide chain of the second antibody are outside the hinge region.

Accordingly, in a further aspect the invention provides a co-delivery system comprising one or more nucleic acid molecules encoding a first antibody and a second antibody, wherein each of the first antibody and the second antibody comprises at least a first polypeptide chain and a second polypeptide chain, wherein the first polypeptide chain and the second polypeptide chain of the first antibody are covalently linked by at least one inter-chain disulphide bond within the hinge region, wherein the first polypeptide chain and the second polypeptide chain of the second antibody are covalently linked by at least one inter-chain disulphide bond, and wherein all of the inter-chain disulphide bonds between the first polypeptide chain and the second polypeptide chain of the second antibody are outside the hinge region.

As used herein, the term "co-delivery system" refers to a single system that enables the co-delivery of two or more therapeutic and/or diagnostic agents into a single cell. In the context of the present invention, the therapeutic and/or diagnostic agents are nucleotide-encoded antibodies. Thus, the co-delivery system of the invention enables the co-expression of multiple antibodies in a single cell.

Many different antibody formats are known in the art. Any antibody for use according to the invention may be any antibody format known in the art. Known antibody formats include, but are not limited to, full-length immunoglobulins (e.g. an IgG antibody), IgG-derived antibodies, VHH-CH3 fusions, minibodies, ΔCH2-IgGs and VHH-Fcs.

As used herein, the term "antibody format" refers to a structural arrangement of an antibody (e.g. a construct) comprising one or more immunoglobulin domains. Said constructs may also be referred to as antigen-specific binding fragments. Suitably, an antibody formant comprises at least one binding moiety (also termed an "antigen binding site"). The remainder of the antibody may comprise any sequences which provide a suitable scaffold for the antigen binding site and display it in an appropriate manner for it to bind the antigen.

Suitably, an antibody for use according to the invention may be a full-length immunoglobulin (e.g. an IgG antibody), a scFv-Fc, a Fab-Fc, a Fab'-Fc, a F(ab)'2-Fc, an Fv-Fc, a sdAb-Fc, a VHH-CH3 fusion, a minibody, a ΔCH2-IgG or a VHH-Fc.

Suitably, an antibody for use according to the invention may be a full-length immunoglobulin, a scFv-Fc, a Fab-Fc, an Fv-Fc, a sdAb-Fc, VHH-CH3 fusion, a minibody, a ΔCH2-IgG or a VHH-Fc.

In some embodiments, the sequence of the antibody may be defined using Kabat numbering (Kabat E.A. et al., (1991)), the EU numbering scheme (Edelman et al., Proc Natl Acad Sci U S A., 1969, 63: 78-85) or the IMGT numbering system (https://www.imgt.org/). The IMGT system is well-known in the art (see, for example, Lefrance et al.; 2003; Dev Comp Immunol; 27: 55-77).

### Disulphides

In the context of the co-delivery system of the invention, each of the first antibody and the second antibody comprises at least a first polypeptide chain and a second polypeptide chain covalently linked by at least one inter-chain disulphide bond, wherein all of the inter-chain disulphide bonds between the first polypeptide chain and the second polypeptide chain of the first antibody are at different positions to the inter-chain disulphide bonds between the first polypeptide chain and the second polypeptide chain of the second antibody.

In one embodiment, the first polypeptide chain and second polypeptide chain of the first antibody are covalently linked by at least two, at least three, at least four, at least five, at least six, at least seven, or at least eight inter-chain disulphide bonds.

In one embodiment, the first polypeptide chain and second polypeptide chain of the second antibody are covalently linked by at least two, at least three, at least four, at least five, at least six, at least seven, or at least eight inter-chain disulphide bonds.

In one embodiment, the first polypeptide chain and second polypeptide chain of the first antibody are covalently linked by one, two, three, four, five, six, seven, or eight inter-chain disulphide bonds.

In one embodiment, the first polypeptide chain and second polypeptide chain of the first antibody are covalently linked by two inter-chain disulphide bonds.

In one embodiment, the first polypeptide chain and second polypeptide chain of the first antibody are covalently linked by three inter-chain disulphide bonds.

In one embodiment, the first polypeptide chain and second polypeptide chain of the second antibody are covalently linked by one, two, three, four, five, six, seven, or eight inter-chain disulphide bonds.

In one embodiment, the first polypeptide chain and second polypeptide chain of the second antibody are covalently linked by one inter-chain disulphide bond. Suitably, the inter-chain disulphide bond is an asymmetric disulphide bond as described herein.

Herein, the terms "disulphide bridge" and "disulphide bond" are used interchangeably.

By "at different positions" is meant that the disulphide bonds in the respective first polypeptide chains or the respective second polypeptide chains of two antibodies that link the first and second polypeptide chains of each antibody are located at different residue positions as identified using an antibody numbering system as described herein (such as the EU numbering, Kabat or IMGT system). Thus, in embodiments none of the disulphide bonds between the first polypeptide chain and the second polypeptide chain of the first antibody are located at the same residue position as any disulphide bond between the first polypeptide chain and the second polypeptide chain of the second antibody. In embodiments, the same applies to the third antibody and the one or more additional antibodies as described herein. This does not exclude the presence of other inter-chain disulphide bonds at the same position within the first and/or second polypeptide chains of the two antibodies, provided that these disulphide bonds at the same position do not link the first and second polypeptide chains of an antibody. For example, the antibodies may comprise an inter-chain disulphide bond linking a third and/or a fourth polypeptide chain with the first and/or second polypeptide chain, respectively, wherein the inter-chain disulphide bond linking the third and/or a fourth polypeptide chain with the first and/or second polypeptide chain is located at the same position within the different antibodies.

In some embodiments, all of the inter-chain disulphide bonds between the first polypeptide chain and the second polypeptide chain of the first antibody are located within the hinge region.

In some embodiments, the first antibody and the second antibody may comprise inter-chain disulphide bonds located at different positions within the hinge regions of the first and second antibody.

In some alternative embodiments, all of the inter-chain disulphide bonds between the first polypeptide chain and the second polypeptide chain of the second antibody are outside the hinge region. Thus, none of the inter-chain disulphide bonds between the first polypeptide chain and the second polypeptide chain of the second antibody are located within the hinge region of the second antibody.

The hinge region of an antibody may be denoted as described herein.

By "located within the hinge region" it is meant that the cysteine residues forming the inter-chain disulphide bond(s) are at residue positions within the hinge region, i.e. denoted the hinge region as described herein.

By "are outside the hinge region" is meant that the cysteine residues forming the inter-chain disulphide bond(s) are at residue positions that are not located within the hinge region, i.e. are not denoted the hinge region.

In some embodiments, the first polypeptide chain and the second polypeptide chain of the first antibody each comprise one or more of C220, C226 and C229 as identified using the EU numbering system.

In one embodiment, the first polypeptide chain and the second polypeptide chain of the first antibody each comprise C220 as identified using the EU numbering system.

In one embodiment, the first polypeptide chain and the second polypeptide chain of the first antibody each comprise C226 as identified using the EU numbering system.

In one embodiment, the first polypeptide chain and the second polypeptide chain of the first antibody each comprise C229 as identified using the EU numbering system.

In one embodiment, the first polypeptide chain and the second polypeptide chain of the first antibody each comprise C220 and C226 as identified using the EU numbering system.

In one embodiment, the first polypeptide chain and the second polypeptide chain of the first antibody each comprise C220 and C229 as identified using the EU numbering system.

In one embodiment, the first polypeptide chain and the second polypeptide chain of the first antibody each comprise a C226 and C229 as identified using the EU numbering system.

In some preferred embodiments, the first polypeptide chain and the second polypeptide chain of the first antibody each comprise C220, C226 and C229 as identified using the EU numbering system.

In some embodiments, the third polypeptide chain and the fourth polypeptide chain of the first antibody each comprise a C214 as identified using the EU numbering system or the Kabat numbering system.

In some embodiments, the first polypeptide chain and the second polypeptide chain of the first antibody are covalently linked by an inter-chain disulphide bond formed between C226 of each of the first and second polypeptide chains as identified using the EU numbering system.

In some embodiments, the first polypeptide chain and the second polypeptide chain of the first antibody are covalently linked by an inter-chain disulphide bond formed between C229 of each of the first and second polypeptide chains as identified using the EU numbering system.

In some embodiments, the first polypeptide chain and the second polypeptide chain of the first antibody are covalently linked by a first inter-chain disulphide bond formed between C226 of each of the first and second polypeptide chains and by a second inter-chain disulphide bond formed between C229 of each of the first and second polypeptide chains as identified using the EU numbering system.

In some embodiments, the first polypeptide chain and the third polypeptide chain of the first antibody are covalently linked by an inter-chain disulphide bond formed between C220 of the first polypeptide chain and C214 of the third polypeptide chain as identified using the EU numbering system.

In some embodiments, the second polypeptide chain and the fourth polypeptide chain of the first antibody are covalently linked by an inter-chain disulphide bond formed between C220 of the second polypeptide chain and C214 of the fourth polypeptide chain as identified using the EU numbering system.

In some embodiments, the first polypeptide chain and the second polypeptide chain of the first antibody are covalently linked by a first inter-chain disulphide bond formed between C226 of each of the first and second polypeptide chains and by a second inter-chain disulphide bond formed between C229 of each of the first and second polypeptide chains as identified using the EU numbering system, and the first polypeptide chain and the third polypeptide chain of the first antibody are covalently linked by an inter-chain disulphide bond formed between C220 of the first polypeptide chain and C214 of the third polypeptide chain as identified using the EU numbering system, and the second polypeptide chain and the fourth polypeptide chain of the first antibody are covalently linked by an inter-chain disulphide bond formed between C220 of the second polypeptide chain and C214 of the fourth polypeptide chain as identified using the EU numbering system.

In some embodiments, positions 220, 226 and 229 of the first and second polypeptide chains of the first antibody as identified using the EU numbering system correspond to positions 223, 239 and 242, respectively, of the first and second polypeptide chains of the first antibody as identified using the Kabat numbering system.

In some embodiments, position 214 of the third and fourth polypeptide chains of the first antibody as identified using the EU numbering system corresponds to position 214 of the third and fourth polypeptide chains of the first antibody as identified using the Kabat numbering system and to position 126 of the third and fourth polypeptide chains of the first antibody as identified using the IMGT numbering system.

Suitably, if the third and fourth polypeptide chains of the first antibody are lambda light chains then the Kabat or IMGT numbering system is used to identify the residue positions.

Thus, in some embodiments, the first antibody comprises:
a) a first inter-chain disulphide bond formed between C226 of each of the first and second polypeptide chains (i.e. the heavy chains) as identified using the EU numbering system (i.e. at position 239 as identified using the Kabat numbering system);
b) a second inter-chain disulphide bond formed between C229 of each of the first and second polypeptide chains (i.e. the heavy chains) as identified using the EU numbering system (i.e. at C242 as identified using the Kabat numbering system);
c) a third inter-chain disulphide bond formed between C220 of the first polypeptide chain (i.e. one heavy chain) and C214 of the third polypeptide chain (i.e. one light chain) as identified using the EU numbering system (i.e. at C223 of the first polypeptide chain and at C214 of the third polypeptide chain as identified using the Kabat numbering system); and/or
d) a fourth inter-chain disulphide bond formed between C220 of the second polypeptide chain (i.e. one heavy chain) and C214 of the fourth polypeptide chain (i.e. one light chain) as identified using the EU numbering system (i.e. at C223 of the second polypeptide chain and at C214 of the fourth polypeptide chain as identified using the Kabat numbering system).

In some embodiments, the first polypeptide chain of the second antibody comprises C354 as identified using the EU numbering system. In some embodiments, C354 as identified using the EU numbering system corresponds to C10 as identified using the IMGT numbering system in a human CH3 domain.

In some embodiments, the second polypeptide chain of the second antibody comprises C349 as identified using the EU numbering system. In some embodiments, C349 as identified using the EU numbering system corresponds to C5 as identified using the IMGT numbering system in a human CH3 domain.

In some embodiments, the first polypeptide chain of the second antibody comprises C354 as identified using the EU numbering system (i.e. C10 as identified using the IMGT numbering system in a human CH3 domain) and the second polypeptide chain of the second antibody comprises C349 as identified using the EU numbering system (i.e. C5 as identified using the IMGT numbering system in a human CH3 domain).

In some embodiments, the first polypeptide chain and the second polypeptide chain of the second antibody are covalently linked by an inter-chain disulphide bond formed between C354 of the first polypeptide chain as identified using the EU numbering system (i.e. C10 as identified using the IMGT numbering system in a human CH3 domain) and C349 of the second polypeptide chain as identified using the EU numbering system (i.e. C5 as identified using the IMGT numbering system in a human CH3 domain).

In some embodiments, the first antibody comprises:
a) a first inter-chain disulphide bond formed between C226 of each of the first and second polypeptide chains (i.e. the heavy chains) as identified using the EU numbering system (i.e. at position 239 as identified using the Kabat numbering system);
b) a second inter-chain disulphide bond formed between C229 of each of the first and second polypeptide chains (i.e. the heavy chains) as identified using the EU numbering system (i.e. at C242 as identified using the Kabat numbering system);
c) a third inter-chain disulphide bond formed between C220 of the first polypeptide chain (i.e. one heavy chain) and C214 of the third polypeptide chain (i.e. one light chain) as identified using the EU numbering system (i.e. at C223 of the first polypeptide chain and at C214 of the third polypeptide chain as identified using the Kabat numbering system); and/or
d) a fourth inter-chain disulphide bond formed between C220 of the second polypeptide chain (i.e. one heavy chain) and C214 of the fourth polypeptide chain (i.e. one light chain) as identified using the EU numbering system (i.e. at C223 of the second polypeptide chain and at C214 of the fourth polypeptide chain as identified using the Kabat numbering system), and
the second antibody comprises an inter-chain disulphide bond formed between C354 of the first polypeptide chain as identified using the EU numbering system (i.e. C10 as identified using the IMGT numbering system in a human CH3 domain) and C349 of the second polypeptide chain as identified using the EU numbering system (i.e. C5 as identified using the IMGT numbering system in a human CH3 domain).

In preferred embodiments, the antibodies encoded by the co-delivery system include IgG-like antibodies, which must comprise at least the Fc-region of an IgG antibody and a partial hinge region. Preferably, the partial hinge region allows for the formation of two inter-chain disulphide bridges that stabilize the Fc-dimer.

In the context of the present invention, the first and second polypeptide chains of the first, second, third or each one or more additional antibody correspond to the heavy chains (HCs) of the antibody. Various antibody formats, including a full-length immunoglobulin (e.g. an IgG antibody) or an IgG-derived antibody, additionally comprise two light chains (LCs), i.e. a full-length immunoglobulin (e.g. an IgG antibody) or an IgG-derived antibody comprises four polypeptide chains: two HCs and two LCs. Preferably, each HC is covalently linked to a LC via a disulphide bond (see Figure 1). For example, the disulphide bond that links the HC with the LC is located at the same position within each HC the antibody. The disulphide bond that links the HC with the LC may be located at the same position within each LC of the antibody. In some embodiments, the first antibody and/or the second antibody further comprises a third polypeptide chain and a fourth polypeptide chain.

In one embodiment, the first antibody further comprises a third polypeptide chain and a fourth polypeptide chain.

In one embodiment, the second antibody further comprises a third polypeptide chain and a fourth polypeptide chain.

In one embodiment, the third polypeptide chain is covalently linked by an inter-chain disulphide bond to the first polypeptide chain of the antibody.

In one embodiment, the fourth polypeptide chain is covalently linked by an inter-chain disulphide bond to the second polypeptide chain of the antibody.

In one embodiment, the inter-chain disulphide bond linking the first polypeptide chain and the third polypeptide chain is at the same position within the first polypeptide chain as compared to the position within the second polypeptide chain of the inter-chain disulphide bond linking the second polypeptide chain and the fourth polypeptide chain.

In one embodiment, the inter-chain disulphide bond linking the first polypeptide chain and the third polypeptide chain is at the same position within the third polypeptide chain as compared to the position within the fourth polypeptide chain of the inter-chain disulphide bond linking the second polypeptide chain and the fourth polypeptide chain.

### Nucleic acid molecule

The co-delivery system of the invention comprises one or more nucleic acid molecules encoding a first antibody and a second antibody as described herein.

In one embodiment, the co-delivery system comprises one, two, three, four, five, six, seven, eight, nine or ten nucleic acid molecule(s).

In one embodiment, the co-delivery system comprises one nucleic acid molecule.

According to the present disclosure, terms such as "capable of expressing", "nucleic acid (molecule) expressing" and "nucleic acid (molecule) encoding" or similar terms are used interchangeably herein and with respect to a particular peptide or polypeptide mean that the nucleic acid, if present in the appropriate environment, e.g. within a cell, can be expressed to produce said peptide or polypeptide.

Suitably, the nucleic acid molecule(s) capable of expressing the antibodies for use according to the invention may comprise a plurality of nucleic acid sequences which encode components of the antibodies such as the first polypeptide chain, second polypeptide chain, Fc region, variable domain, VHH domain, and/or linking sequence as described herein.

In one embodiment, each nucleic acid molecule comprises one or more nucleotide sequences encoding a polypeptide chain of an antibody. Thus, in some embodiments, each nucleic acid molecule encodes at least one (suitably, at least two, at least three, at least four, at least five or at least six) polypeptide chain(s) of an antibody.

It will be understood by the skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described herein to reflect the codon usage of any particular host organism in which the polypeptides are to be expressed. Suitably, the polynucleotides of the present invention are codon optimised to enable expression in a mammalian cell, in particular a cell as described herein.

Nucleic acids according to the invention may comprise DNA and/or RNA. The nucleic acids may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. Herein, it is to be understood that the polynucleotides may be modified by any method available in the art. Such modifications may be carried out in order to enhance the in vivo activity or life span of polynucleotides of interest.

In some embodiments, the nucleic acid molecule(s) is DNA and/or RNA.

In one embodiment, the nucleic acid molecule(s) is DNA.

In one embodiment, the nucleic acid molecule(s) is RNA.

### Antibodies

The co-delivery system of the invention comprises one or more nucleic acid molecules encoding a first antibody and a second antibody.

The one or more nucleic acid molecules may further encode a third antibody or one or more additional antibodies as described herein.

As used herein, the term "antibody" may refer to a protein or polypeptide having an antigen-binding domain which comprises at least one complementarity determining region (CDR). The term "complementarity determining region" or "CDR" generally refers to one of the 6 hypervariable regions within the variable domain of an antibody.

"Complementarity determining region" or "CDR" with regard to an antigen-binding domain or antibody refers to a hypervariable region or a highly variable loop in the variable region of the heavy chain of the light chain of an antibody, which contribute primarily to antigen binding. CDRs can interact with the antigen conformation and largely determine binding to the antigen (although some framework regions are known to be involved in binding). The heavy chain variable region and the light chain variable region each contain 3 CDRs (heavy chain CDRs 1, 2 and 3 and light chain CDRs 1, 2 and 3, numbered from the amino to the carboxy terminus).

Techniques for preparing and using various antibody-based constructs and fragments are well known in the art.

As used herein, "antigen binding site" or "antigen-binding domain" means a protein or polypeptide which comprises at least one complementarity determining region (CDR). The antigen binding site may comprise 3 CDRs, i.e. be equivalent to that of a single domain antibody (sdAb) domain such as a VHH domain.

As used herein, the term "hinge region" refers to the region of the native immunoglobulin chain which is denoted the hinge region. For example, the hinge region may be denoted according to the Kabat numbering scheme (Kabat E.A. et al., (1991)) or the EU numbering scheme (Edelman et al., Proc Natl Acad Sci U S A., 1969, 63: 78-85). For example, the IgG1 hinge region according to EU numbering scheme is from amino acid (AA) 216-230, and according to Kabat numbering scheme is from AA 226-243.

The components of the antibodies may be connected by a linking sequence. The linking sequence may comprise one or more linkers (e.g. glycine-serine (GS) linkers), such as those linkers that are widely known in the art. In an embodiment, the linking sequence comprises a GS linker. In an embodiment, the linking sequence consists of a GS linker. In an embodiment, the linking sequence is GS. In a preferred embodiment, the linking sequence is devoid of cysteine residues. Thus, the linking sequence cannot form intra- or inter-chain disulphide bonds.

As used herein, the term "constant immunoglobulin domain" may refer to a constant domain of an immunoglobulin, e.g. a CH3 domain. Suitably, the constant immunoglobulin domain (e.g. CH3 domain) may be an IgG1, IgG2, IgG3, IgG4, IgA, IgE, IgM or IgD constant domain. Suitably, the constant immunoglobulin domain (e.g. CH3 domain) may be an IgG1, IgG2, IgG3 or IgG4 constant domain. In a preferred embodiment, the constant immunoglobulin domain (e.g. CH3 domain) is an IgG1 constant domain. Suitably, the constant immunoglobulin domain may be an IgE or IgM constant domain (e.g. CH4 domain).

In conventional, full-length antibodies (e.g. IgG antibodies) that comprise four polypeptides - two light chains and two heavy chains - the Fc region forms a homodimer of the CH2-CH3 domains of each heavy chain polypeptide.

As used herein, the term "fragment crystallisable (Fc) region" may refer to the Fc region of an immunoglobulin, which comprises the CH2-CH3 domains. Suitably, in embodiments of the invention, the Fc region may be an IgG1, IgG2, IgG3, IgG4, lgA1, IgA2, IgE, IgM or IgD Fc region. Suitably, in embodiments of the invention, the Fc region may be an IgG1, IgG2, IgG3 or IgG4 Fc region. In a preferred embodiment, the Fc region is an IgG1 Fc region. In the context of the present invention, the first polypeptide and the second polypeptide of a single antibody each comprise an Fc region of the same immunoglobulin isotype.

Any suitable Fc region which is known in the art may be employed in the practice of the present invention. In some embodiments, one or more amino acid modifications may be introduced into an Fc region of an antibody provided herein, thus producing an Fc region variant. The Fc region variant may comprise a human Fc region sequence (such as the human IgG1, IgG2, IgG3, or IgG4 Fc region) comprising an amino acid modification (such as substitution) at one or more amino acid positions. For example, Fc engineering for modulated effector functions is known in the art. Fc modifications include, but are not limited to, silencing effector functions, elevating effector functions, and elongating or shortening half-life. A review of Fc modifications is provided by Liu et al. (Liu et al., Antibodies, 2020, 9: 64).

sdAbs are IgG molecules that are found naturally in e.g. camelids and sharks. Camelid sdAbs are devoid of the light chain and lack the first constant domain of the heavy chain (CH1) of conventional IgGs. Consequently, the antigen-binding fragment of sdAbs solely comprises a single variable domain, often referred to as a Variable Heavy domain of Heavy chain (VHH domain) (also referred to herein as a "single domain variable heavy immunoglobulin" domain). Thus, a VHH comprises 3 CDRs.

As used herein, the term "variable immunoglobulin domain" or "variable domain" may refer to a variable domain of an immunoglobulin, e.g. a VHH domain.

The immunoglobulin domains used in the invention are not limited to a specific sequence, and may comprise any suitable known immunoglobulin domains.

The first polypeptide and/or the second polypeptide of each antibody for use according to the invention comprises at least one binding moiety, which may be a variable domain.

In some embodiments, the at least one variable domain is selected from the group consisting of a single-chain variable fragment (scFv); an Fab; an Fab'; an F(ab)'2; an Fv; a single domain antibody (sdAb); a VHH; a single chain variable domain; a designed ankyrin repeat protein (DARPin); and an aptamer.

In some embodiments, the at least one variable domain is selected from the group consisting of a single-chain variable fragment (scFv); an Fab; an Fv; a single domain antibody (sdAb); a VHH; a single chain variable domain; and a designed ankyrin repeat protein (DARPin).

In one embodiment, the at least one variable domain of the first polypeptide is a variable domain of the same type as the at least one variable domain of the second polypeptide. For example, the variable domain of the first polypeptide may be a VHH and the variable domain of the second polypeptide may be a VHH.

In one embodiment, the at least one variable domain of the first polypeptide is of a different type to the at least one variable domain of the second polypeptide. For example, the variable domain of the first polypeptide may be an scFv and the variable domain of the second polypeptide may be a VHH. Suitably, an antibody for use according to the invention may be designed using CrossMAb technology (Schaefer et al., PNAS, 2011, 108: 11187-92; Takahashi et al., Cell, 1982, 29: 671-679; Grunert et al., ACS Omega 2022, 7, 4, 3671-3679; and Klein et al., mAbs, 2016, 8: 1010-1020).

### Binding moiety and cognate of a binding moiety

Each polypeptide chain of an antibody may comprise at least one binding moiety or at least one cognate of a binding moiety at the N- and/or C-terminus. Suitably, either the N- or C-terminus may be left free.

In some embodiments, the first polypeptide chain of the first antibody comprises at least one binding moiety or at least one cognate of a binding moiety at the N- and/or C-terminus.

In some embodiments, the second polypeptide chain of the first antibody comprises at least one binding moiety or at least one cognate of a binding moiety at the N- and/or C-terminus.

In some embodiments, the first polypeptide chain of the first antibody comprises at least one binding moiety or at least one cognate of a binding moiety at the N- and/or C-terminus and the second polypeptide chain of the first antibody comprises at least one binding moiety or at least one cognate of a binding moiety at the N- and/or C-terminus.

In one embodiment, the first polypeptide chain of the first antibody comprises at least one binding moiety at the N-terminus.

In one embodiment, the first polypeptide chain of the first antibody comprises at least one binding moiety at the C-terminus.

In one embodiment, the first polypeptide chain of the first antibody comprises at least one binding moiety at the N-terminus and at least one binding moiety at the C-terminus.

In one embodiment, the first polypeptide chain of the first antibody comprises at least one cognate of a binding moiety at the N-terminus.

In one embodiment, the first polypeptide chain of the first antibody comprises at least one cognate of a binding moiety at the C-terminus.

In one embodiment, the first polypeptide chain of the first antibody comprises at least one cognate of a binding moiety at the N-terminus and at least one cognate of a binding moiety at the C-terminus.

In one embodiment, the first polypeptide chain of the first antibody comprises at least one binding moiety at the N-terminus and at least one cognate of a binding moiety at the C-terminus.

In one embodiment, the first polypeptide chain of the first antibody comprises at least one binding moiety at the C-terminus and at least one cognate of a binding moiety at the N-terminus.

In one embodiment, the second polypeptide chain of the first antibody comprises at least one binding moiety at the N-terminus.

In one embodiment, the second polypeptide chain of the first antibody comprises at least one binding moiety at the C-terminus.

In one embodiment, the second polypeptide chain of the first antibody comprises at least one binding moiety at the N-terminus and at least one binding moiety at the C-terminus.

In one embodiment, the second polypeptide chain of the first antibody comprises at least one cognate of a binding moiety at the N-terminus.

In one embodiment, the second polypeptide chain of the first antibody comprises at least one cognate of a binding moiety at the C-terminus.

In one embodiment, the second polypeptide chain of the first antibody comprises at least one cognate of a binding moiety at the N-terminus and at least one cognate of a binding moiety at the C-terminus.

In one embodiment, the second polypeptide chain of the first antibody comprises at least one binding moiety at the N-terminus and at least one cognate of a binding moiety at the C-terminus.

In one embodiment, the second polypeptide chain of the first antibody comprises at least one binding moiety at the C-terminus and at least one cognate of a binding moiety at the N-terminus.

In some embodiments, the first polypeptide chain of the second antibody comprises at least one binding moiety or at least one cognate of a binding moiety at the N- and/or C-terminus.

In some embodiments, the second polypeptide chain of the second antibody comprises at least one binding moiety or at least one cognate of a binding moiety at the N- and/or C-terminus.

In some embodiments, the first polypeptide chain of the second antibody comprises at least one binding moiety or at least one cognate of a binding moiety at the N- and/or C-terminus and the second polypeptide chain of the second antibody comprises at least one binding moiety or at least one cognate of a binding moiety at the N- and/or C-terminus.

In one embodiment, the first polypeptide chain of the second antibody comprises at least one binding moiety at the N-terminus.

In one embodiment, the first polypeptide chain of the second antibody comprises at least one binding moiety at the C-terminus.

In one embodiment, the first polypeptide chain of the second antibody comprises at least one binding moiety at the N-terminus and at least one binding moiety at the C-terminus.

In one embodiment, the first polypeptide chain of the second antibody comprises at least one cognate of a binding moiety at the N-terminus.

In one embodiment, the first polypeptide chain of the second antibody comprises at least one cognate of a binding moiety at the C-terminus.

In one embodiment, the first polypeptide chain of the second antibody comprises at least one cognate of a binding moiety at the N-terminus and at least one cognate of a binding moiety at the C-terminus.

In one embodiment, the first polypeptide chain of the second antibody comprises at least one binding moiety at the N-terminus and at least one cognate of a binding moiety at the C-terminus.

In one embodiment, the first polypeptide chain of the second antibody comprises at least one binding moiety at the C-terminus and at least one cognate of a binding moiety at the N-terminus.

In one embodiment, the second polypeptide chain of the second antibody comprises at least one binding moiety at the N-terminus.

In one embodiment, the second polypeptide chain of the second antibody comprises at least one binding moiety at the C-terminus.

In one embodiment, the second polypeptide chain of the second antibody comprises at least one binding moiety at the N-terminus and at least one binding moiety at the C-terminus.

In one embodiment, the second polypeptide chain of the second antibody comprises at least one cognate of a binding moiety at the N-terminus.

In one embodiment, the second polypeptide chain of the second antibody comprises at least one cognate of a binding moiety at the C-terminus.

In one embodiment, the second polypeptide chain of the second antibody comprises at least one cognate of a binding moiety at the N-terminus and at least one cognate of a binding moiety at the C-terminus.

In one embodiment, the second polypeptide chain of the second antibody comprises at least one binding moiety at the N-terminus and at least one cognate of a binding moiety at the C-terminus.

In one embodiment, the second polypeptide chain of the second antibody comprises at least one binding moiety at the C-terminus and at least one cognate of a binding moiety at the N-terminus.

In some embodiments, the first polypeptide chain of the third antibody comprises at least one binding moiety or at least one cognate of a binding moiety at the N- and/or C-terminus.

In some embodiments, the second polypeptide chain of the third antibody comprises at least one binding moiety or at least one cognate of a binding moiety at the N- and/or C-terminus.

In some embodiments, the first polypeptide chain of the third antibody comprises at least one binding moiety or at least one cognate of a binding moiety at the N- and/or C-terminus and the second polypeptide chain of the third antibody comprises at least one binding moiety or at least one cognate of a binding moiety at the N- and/or C-terminus.

In one embodiment, the first polypeptide chain of the third antibody comprises at least one binding moiety at the N-terminus.

In one embodiment, the first polypeptide chain of the third antibody comprises at least one binding moiety at the C-terminus.

In one embodiment, the first polypeptide chain of the third antibody comprises at least one binding moiety at the N-terminus and at least one binding moiety at the C-terminus.

In one embodiment, the first polypeptide chain of the third antibody comprises at least one cognate of a binding moiety at the N-terminus.

In one embodiment, the first polypeptide chain of the third antibody comprises at least one cognate of a binding moiety at the C-terminus.

In one embodiment, the first polypeptide chain of the third antibody comprises at least one cognate of a binding moiety at the N-terminus and at least one cognate of a binding moiety at the C-terminus.

In one embodiment, the first polypeptide chain of the third antibody comprises at least one binding moiety at the N-terminus and at least one cognate of a binding moiety at the C-terminus.

In one embodiment, the first polypeptide chain of the third antibody comprises at least one binding moiety at the C-terminus and at least one cognate of a binding moiety at the N-terminus.

In one embodiment, the second polypeptide chain of the third antibody comprises at least one binding moiety at the N-terminus.

In one embodiment, the second polypeptide chain of the third antibody comprises at least one binding moiety at the C-terminus.

In one embodiment, the second polypeptide chain of the third antibody comprises at least one binding moiety at the N-terminus and at least one binding moiety at the C-terminus.

In one embodiment, the second polypeptide chain of the third antibody comprises at least one cognate of a binding moiety at the N-terminus.

In one embodiment, the second polypeptide chain of the third antibody comprises at least one cognate of a binding moiety at the C-terminus.

In one embodiment, the second polypeptide chain of the third antibody comprises at least one cognate of a binding moiety at the N-terminus and at least one cognate of a binding moiety at the C-terminus.

In one embodiment, the second polypeptide chain of the third antibody comprises at least one binding moiety at the N-terminus and at least one cognate of a binding moiety at the C-terminus.

In one embodiment, the second polypeptide chain of the third antibody comprises at least one binding moiety at the C-terminus and at least one cognate of a binding moiety at the N-terminus.

As used herein, the term "binding moiety" refers to any suitable binding entity which is capable of specifically binding to a target, such as a target polypeptide sequence. Numerous binding moieties are known in the art, including those based on the antigen binding site of an antibody, antibody mimetics, and T-cell receptors. For example, the binding moiety may comprise: a variable immunoglobulin domain; an antigen binding site of an antibody; a single-chain variable fragment (scFv); a Fab; an Fv; a single domain antibody (sdAb); a VHH; a single chain variable domain (which may be a VH or VL chain, having 3 CDRs); or an artificial single binder such as a Darpin (designed ankyrin repeat protein). In a preferred embodiment, the binding moiety is an antigen-binding fragment of an antibody. In a preferred embodiment, the binding moiety is a VHH.

As used herein, the term "cognate of a binding moiety" refers to any motif that is specifically recognised by a binding moiety. Numerous cognates of binding moieties are provided in the art. In an embodiment, the cognate is an antigen, an epitope or a polypeptide tag that is specifically recognised by an antibody or an antigen-binding fragment of an antibody. In a preferred embodiment, the cognate is a polypeptide tag. In a preferred embodiment, the cognate is a polypeptide tag that is specifically recognised by an antigen-binding fragment of an antibody.

In an embodiment, the cognate of a binding moiety is not specifically recognised by any of the variable domains (e.g. VHH domains) and/or binding moieties of any of the antibodies delivered in the co-delivery system of the invention.

In an embodiment, a binding moiety or a variable domain (e.g. a VHH) "specific for" a target or "specifically recognising" a target refers to binding to the target with an affinity equivalent to that of a functional antibody fragment. In an embodiment, the targets are target antigens.

In one embodiment, the binding moieties of the first and the second polypeptide of any antibody as described herein have the same binding specificity.

In one embodiment the binding moieties of the first and the second polypeptide of any antibody as described herein have different binding specificities.

In an embodiment in which the first and/or second polypeptides contain a plurality of variable domains and/or binding moieties (e.g. VHH domains), each variable domain and/or binding moiety (e.g. VHH domain) in the same polypeptide may be specific for the same target. In an embodiment in which the first and/or second polypeptides contain a plurality of variable domains and/or binding moieties (e.g. VHH domains), each variable domain and/or binding moiety (e.g. VHH domain) in the same polypeptide may be specific for a different target.

In an embodiment, for a given antibody, the variable domain(s) (e.g. VHH domain(s)) at the N terminus of the first polypeptide are specific for a first target and the variable domain(s) (e.g. VHH domain(s)) at the N terminus of the second polypeptide are specific for a second target. In a preferred embodiment, the first and second targets are different. In an embodiment, accordingly, the antibody comprising the first and second polypeptides is a heterodimer. In an alternative embodiment, for a given antibody, the variable domain(s) (e.g. VHH domain(s)) at the N terminus of the first polypeptide are specific for the same target as the variable domain(s) (e.g. VHH domain(s)) at the N terminus of the second polypeptide. In an embodiment, accordingly, the antibody comprising the first and second polypeptides is a homodimer.

In an embodiment, for a given antibody, the variable domain(s) (e.g. VHH domain(s)) at the C terminus of the first polypeptide and/or the second polypeptide is preferably a VHH domain and is specific for a third target. In an embodiment, the third target is different to the first and second targets. In an embodiment, for a given antibody, the variable domain(s) (e.g. VHH domain(s)) at the C terminus of each of the first polypeptide and the second polypeptide are preferably VHH domains and are specific for a third target and a fourth target. In an embodiment, each of the first, second, third and fourth targets are different.

The binding moieties (e.g. variable domains such as VHHs) used in the invention are not particularly limiting, and may be specific for any selected target protein. The selected target protein may be a cancer antigen, an immune cell marker, or a non-human cell antigen.

In some embodiments, for any given antibody, the binding moiety of the first polypeptide, the second polypeptide or both the first polypeptide and the second polypeptide is specific for a cancer antigen.

Various tumour associated antigens (TAA) are known in the art. In an embodiment, the variable domains (e.g. VHH domains) of the present invention are capable of specifically binding to a TAA.

In some embodiments, for any given antibody, the binding moiety of the first polypeptide, the second polypeptide or both the first polypeptide and the second polypeptide is specific for an immune cell marker. Various immune cell markers are known in the art. For example, anti-CD163 antibodies that target macrophages are known in the art (Liu et al., Expert Opinion on Biological Therapy, 2016, 16: 591-593).

By way of further example, antibodies targeting CXCR4 are known in the art. CXCR4 is highly expressed on T cells, B-cells, and monocytes, as well as hematopoietic stem cells, with minimal to no expression on non-hematopoietic cells (Liu et al., Expert Opinion on Biological Therapy, 2016, 16: 591-593).

Moreover, antibodies targeting non-human cells are known in the art. For example, an antibody-antibiotic conjugate (AAC) targeting intracellular *Streptococcus aureus* has been reported (Liu et al., Expert Opinion on Biological Therapy, 2016, 16: 591-593).

### Antibody types

In some embodiments, the first and second antibodies are independently selected from the group consisting of a human antibody, a humanized antibody, a chimeric antibody, a multispecific antibody, a monoclonal antibody, a polyclonal antibody, or any combination thereof.

The term "chimeric antibody" generally refers to an antibody obtained by fusing a variable region of a non-human antibody and a constant region of a human antibody, which can reduce an immune response induced by the non-human antibody. The non-human antibody may be, for example, a murine, camelid, rabbit, sheep, goat or chicken antibody. By way of example, for establishment of a chimeric antibody, a hybridoma secreting a specific monoclonal antibody can be established, and a variable region gene is cloned from the mouse hybridoma cells; then a constant region gene of human antibody can be cloned as required, and the mouse variable region gene and the human constant region gene are connected to form a chimeric gene; then the chimeric gene is inserted into an expression vector, wherein chimeric antibody molecules can be expressed in a eukaryotic system or a prokaryotic system.

The term "humanized antibody", also referred to as CDR-grafted antibody, generally refers to an antibody produced by grafting mouse CDR sequences into a human antibody variable region framework, i.e., an antibody produced in a different type of human germline antibody framework sequence. Therefore, the heterogeneous reaction induced by the presence of a large number of mouse protein components in the chimeric antibody can be overcome. Such framework sequences can be obtained from public DNA databases or disclosed references that include germline antibody gene sequences. For example, germline DNA sequences of human heavy and light chain variable region genes can be obtained from the "VBase" human germline sequence database.

The term "fully humanized antibody", "fully human antibody" or "completely human antibody", which may also be known as "fully humanized monoclonal antibody", may have both humanized variable region and constant region so as to eliminate immunogenicity and toxic side effects. The development of monoclonal antibodies has four stages, namely murine monoclonal antibodies, chimeric monoclonal antibodies, humanized monoclonal antibodies and fully humanized monoclonal antibodies. The antibodies or ligands described herein can be fully humanized monoclonal antibodies. Relevant technologies for the preparation of fully human antibodies may be: human hybridoma technology, EBV-transformed B-lymphocyte technology, phage display technology, transgenic mouse antibody preparation technology, single B-cell antibody preparation technology, and the like.

A "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

In some embodiments, the first and/or second antibody is a monoclonal antibody.

In some embodiments, each of the first and second antibodies is a monoclonal antibody.

In some embodiments, the first and/or second antibody is a multispecific antibody.

In some embodiments, each of the first and second antibodies is a multispecific antibody.

In some embodiments, the first and/or second antibody is a monoclonal, multispecific antibody.

In some embodiments, each of the first and second antibodies is a monoclonal, multispecific antibody.

Antibodies may be obtained by techniques comprising immunizing an animal with a target antigen and isolating the antibody from serum. Monoclonal antibodies may be made by the hybridoma method first described by Kohler et al., Nature 256:495 (1975), or may be made by recombinant DNA methods (see, e.g., U.S. Pat. No. 4,816,567). Monoclonal antibodies may also be isolated from phage antibody libraries using the techniques described in Clackson et al., Nature 352:624-628 (1991) and Marks et al., J. Mol. Biol. 222:581-597 (1991), for example.

In an embodiment, according to the number of antigen-binding domains, e.g. VHH domains, that are present, the antibody for use according to the present invention may be monovalent, bivalent, trivalent, tetravalent or pentavalent. Similarly, in an embodiment, according to the number of different antigen-binding domains, e.g. VHH domains, that are present, the antibody for use according to the invention may be monospecific or multispecific, e.g., bispecific, trispecific, tetraspecific etc.

Any suitable multispecific or multivalent antibody format which is known in the art may be used in the practice of the present invention. Suitably, the bispecific or bivalent antibody may be an IgG-scFv, IgG-sdAb, IgG-VHH, scFv-Fc-scFv, KiH-IgG, κλ-body, KiH-Fc-Fab/scFv.

The variable domains (e.g. VHH domains) used in the invention are not particularly limiting, and may comprise any antigen binding site that is specific for a selected target protein. Methods for determining binding specificity of an antibody to a particular antigen are known in the art include, but are not limited to, by biolayer interferometry (BLI), surface plasmon resonance (SPR) analysis (e.g. using a BIAcore instrument), ELISA, western blot, *in situ* hybridisation, immunohistochemistry, flow cytometry, Förster resonance energy transfer (FRET), phage display libraries, yeast two-hybrid screens, co-immunoprecipitation, bimolecular fluorescence complementation and tandem affinity purification. Binding affinity can also be determined using methods such as BLI, SPR analysis (e.g. using a BIAcore instrument), flow cytometry, fluorescence quenching, isothermal titration calorimetry.

Methods for providing variable domains, such as sdAbs (which comprise VHH domains), against a specific target are known in the art (see Caussinus et al.; Nat Struct Mol Biol; 2011; 19(1); 117-121 & Fulcher et al.; Open Biol; 2016; 6(10); pii 160255). Further, methods to isolate antigen-specific VHHs from immune or semisynthetic libraries using phage, yeast, or ribosome display are established in the art (see Muyldermans J Biotechnol. 2001 Jun; 74(4):277-302. & Dufner et al. Trends Biotechnol. 2006 Nov; 24(11):523-9).

By way of example, a VHH can be obtained by immunisation of e.g. dromedaries, camels, llamas or alpacas with the desired antigen and subsequent isolation of the mRNA coding for VHHs. Single domain shark variable domain of new antigen receptor (VNAR) antibodies are also known and suitable for use according to the present invention as an alternative sdAb to a VHH domain. Hence, by way of further example, a VNAR can be obtained by immunisation of sharks with the desired antigen and subsequent isolation of the mRNA coding for VNARs. Reverse transcription and PCR can then be used to generate a library of VHHs or VNARs. Standard screening techniques such as phage display and ribosome display may be used to identify the suitable clones binding the antigen of interest.

Once the most potent clones have been identified, their DNA sequence may be optimized, for example to improve their stability towards enzymes. Humanisation may also be performed.

VHHs and VNARs may be expressed in a cell using conventional vectors, such as those described herein.

The ability of the antibody to specifically bind its target may be determined using methods which are known in the art. For example, determination of binding may be performed e.g. by biolayer interferometry (BLI), surface plasmon resonance (SPR) analysis (using a BIAcore instrument), western blot, flow cytometry, *in situ* hybridisation and/or microscopy. Suitably, determination of binding affinity may be performed by e.g. by biolayer interferometry (BLI), surface plasmon resonance (SPR) analysis (using a BIAcore instrument) and/or flow cytometry. Suitably, determination of binding affinity may be performed as described herein (see Example 3).

### IgG and VHH-CH3 fusion

In some embodiments, the first antibody comprises an Fc region.

In some embodiments, the first antibody is an IgG antibody.

In one embodiment, the first antibody is an IgG1 antibody. In one embodiment, the first antibody is an IgG2 antibody. In one embodiment, the first antibody is an IgG3 antibody. In one embodiment, the first antibody is an IgG4 antibody.

In some embodiments, the first antibody is an IgG-derived antibody. Suitably, an IgG-derived antibody may be an IgG antibody that has been modified as described herein. For example, the IgG-derived antibody may comprise a truncated hinge region, may comprise an Fc region variant, and/or may be trivalent or tetravalent (i.e. may comprise one or more binding moieties at the C-terminus).

In some embodiments, the first polypeptide chain and the second polypeptide chain of the second antibody each further comprises one or more constant heavy 3 (CH3) domains.

Suitably, the CH3 domain may be an IgG1, IgG2, IgG3, IgG4, IgA, IgE, IgM or IgD CH3 domain. Suitably, the CH3 domain may be an IgG1, IgG2, IgG3 or IgG4 constant domain. In a preferred embodiment, the CH3 domain is an IgG1 constant domain.

An illustrative human IgG1 CH3 sequence is provided below:

GQPREPQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 1).

In one embodiment, each CH3 domain of the first polypeptide chain and of the second polypeptide chain comprises or consists of a sequence as set forth in SEQ ID NO: 1, or a sequence having 80%, 85%, 90%, 95%, 96%, 97%, 98%, 99% or 100% identity thereto. In an embodiment, the sequences of the CH3 domains further comprise a modification that enhances the formation of the construct comprising the first and second polypeptide chain as defined herein.

In an embodiment, the first polypeptide chain and the second polypeptide chain of the second antibody do not contain any constant immunoglobulin domain(s) other than a CH3 domain. Thus, the first polypeptide chain and the second polypeptide chain of the second antibody do not contain a CH 1, CH2, or CH4 domain.

In a typical and highly preferred embodiment, the first polypeptide chain of the second antibody comprises a single CH3 domain and the second polypeptide chain of the second antibody comprises a single CH3 domain. In an embodiment, the first and/or second polypeptide chain of the second antibody each comprise one CH3 domain. In an embodiment, the first and/or second polypeptide chain of the second antibody each comprise no more than one CH3 domain. In an embodiment, the first and/or second polypeptide chain of the second antibody each comprise fewer than two CH3 domains.

In an alternative embodiment, the first and/or second polypeptide chain of the second antibody comprises more than one CH3 domain. In an embodiment, the CH3 domains of each polypeptide are adjacent, preferably immediately adjacent. In an embodiment comprising more than one CH3 domain it will be understood that, e.g., the first and/or second polypeptide chains of the second antibody do not comprise a variable domain in between the CH3 domains.

In an embodiment, any given antibody for use according to the invention is a dimer insofar as the antibody comprises two polypeptide chains (the first polypeptide chain and the second polypeptide chain) as defined herein. Accordingly, in an embodiment, any given antibody for use according to the invention may further comprise additional elements such as one or more conjugates, but the antibody is still considered a dimer. In an embodiment, the antibody is a homodimer. In a preferred embodiment, the antibody is a heterodimer.

In one embodiment, the first antibody is a homodimer.

In one embodiment, the second antibody is a heterodimer.

In an embodiment, the first and second polypeptide chains of the second antibody comprise modifications that enhance the formation of a dimer, preferably a heterodimer, comprising one monomer of the first polypeptide chain and one monomer of the second polypeptide chain. In an embodiment, the modifications can be those modifications which are widely known in the art of antibody technology for the purpose of (hetero)dimerisation between antibody chains (e.g. "knobs-into-holes" (KiH) modifications). In a typical embodiment, the modifications are present in the CH3 domains of the first and second polypeptide chains of the second antibody.

In one embodiment, one or more of the CH3 domains of the first polypeptide chain and/or one or more of the CH3 domains of the second polypeptide chain of the second antibody comprise a modification that enhances the formation of the dimeric antibody comprising the first and second polypeptide chain. In an embodiment wherein the first and second polypeptide chains each comprise more than one CH3 domain, all of the CH3 domains may comprise such modifications.

In some embodiments, one or more of the CH3 domains of the first polypeptide chain and/or one or more of the CH3 domains of the second polypeptide chain of the second antibody comprise a modification that enhances the formation of the antibody.

As used herein, the term "enhances the formation of the antibody" includes promoting dimerisation of the first and second polypeptide chains. Thus, in an embodiment, the modifications promote dimerisation of the first and second polypeptide chains. In an embodiment, the modifications promote heterodimerisation of the first and second polypeptide chains. By heterodimerisation it is meant that the first polypeptide chain is different to the second polypeptide chain, and the first polypeptide chain will form a dimer with the second polypeptide chain, but the first polypeptide chain will not form a dimer with the first polypeptide chain and the second polypeptide chain will not form a dimer with the second polypeptide chain. In an embodiment, the modifications promote homodimerisation of the first and second polypeptide chains. By homodimerisation it is meant that the first polypeptide chain is the same as the second polypeptide chain, and the first polypeptide chain will form a dimer with the second polypeptide chain.

Various means of promoting dimerisation of two domains (e.g. homo- or hetero-dimerisation) are known in the art. In an embodiment, the two domains may comprise a modification that enhances formation of dimer, e.g. a modification that increases the affinity of the two domains, promotes and/or enables the formation of one or more disulphide bonds, reduces steric hindrance to the dimerisation, promotes electrostatic and/or hydrophilic/hydrophobic interactions between the two domains, or any combination thereof.

In some embodiments, one or more of the CH3 domains of the first polypeptide chain and/or one or more of the CH3 domains of the second polypeptide chain of the second antibody comprise a modification that enhances the affinity between the first and second polypeptide chains. In an embodiment, the modification enhances the affinity between the CH3 domains of the first and second polypeptide chains.

In some embodiments, one or more of the CH3 domains of the first polypeptide chain and/or one or more of the CH3 domains of the second polypeptide chain of the second antibody comprise a modification that reduces steric hindrance to the formation of the antibody. In an embodiment, the modification reduces steric hindrance between the CH3 domains of the first and second polypeptide chains.

In some embodiments, one or more of the CH3 domains of the first polypeptide chain and one or more of the CH3 domains of the second polypeptide chain of the second antibody are associated via a 'knobs-into-holes' modification. In an embodiment, one or more of the CH3 domains of the first polypeptide chain and one or more of the CH3 domains of the second polypeptide chain comprise a 'knobs-into-holes' modification.

In one embodiment, one or more of the CH3 domains of the first polypeptide chain and/or one or more of the CH3 domains of the second polypeptide chain of the second antibody comprise a modification that promotes electrostatic interactions between the first and second polypeptide chains. In an embodiment, the modification promotes electrostatic interactions between the CH3 domains of the first and second polypeptide chains. In an embodiment, the electrostatic interactions that are promoted are favourable to the formation of a dimer (e.g. a heterodimer) comprising the first and second polypeptide chains.

In one embodiment, one or more of the CH3 domains of the first polypeptide chain and/or one or more of the CH3 domains of the second polypeptide chain comprise a modification that promotes hydrophilic/hydrophobic interactions between the first and second polypeptide chains. In an embodiment, the modification promotes hydrophilic/hydrophobic interactions between the CH3 domains of the first and second polypeptide chains. In an embodiment, the hydrophilic/hydrophobic interactions that are promoted are favourable to the formation of a dimer comprising the first and second polypeptide chains.

Any suitable technology for promoting the dimerisation of CH3 domains, i.e. of at least one of the CH3 domains of the first polypeptide chain and at least one of the CH3 domains of the second polypeptide chain, may be employed in the practice of the invention.

Preferably, the modification that enhances the formation of a dimer between the CH3 domains is KiH technology (Ridgeway et al., Protein Engineering, Design and Selection, 1996, 9: 617-621 and Merchant et al., Nat Biotechnol, 1998, 16: 677-681). The KiH technology is based on an engineered pair of CH3 domains that heterodimerises (CH3 heterodimer) in which asymmetric hydrophobic mutations are introduced between the homodimeric CH3 domain. KiH involves introducing mutations that create a protuberance ("knob") in the interface of the first CH3 domain and a corresponding cavity ("hole") in the interface of the second CH3 domain, such that the protuberance can be positioned in the cavity to promote heterodimer assembly and hinder homodimer formation. KiH variants therefore thermodynamically favour the formation of heterodimers rather than homodimers.

By way of further example, the present invention may employ: the strand-exchange engineered domain (SEED) CH3 dimers (Davis et al., Protein Eng Des Sel., 2010, 23:195-202); electrostatic steering employing DD-KK variants with asymmetric electrostatic interactions (Gunasekaran et al., J Biol Chem., 2010 , 285: 19637-46): Azymetric technologies by Zymeworks (https://www.zymeworks.com/technologies/azymetric/): Bispecific Engagement by Antibodies based on the T-cell receptor (BEAT) (Skegro et al., J Biol Chem., 2017, 292: 9745-9759): Fast-Ig and ART-Ig (https://www.chugaipharm.co.jp/english/profile/rd/technologies.html): DEKK dimerisation technology (https://merus.nl/technology/multiclonics-platform/ and Nardis et al, J Biol Chem., 2017, 292: 14706-14717): HA-TF variants with asymmetric hydrophobic interactions (Moore et al., MAbs, 2011, 3: 546-57): computationally designed CH3 interfaces, such as the 7.8.60 design (Leaver-Fay et al., Structure, 2016, 24: 641-651): EW-RVT variants, which were designed to replace the conserved electrostatic interactions with asymmetric hydrophobic interactions and to add asymmetric long-range electrostatic interactions at the rim of the heterodimeric CH3 interface (Choi et al., Mol Cancer Ther., 2013, 12: 2748-59): and the K370E_{CH3A}-E357N_{CH3B} mutations employed in the "A107" variant which replaced the homodimer-favouring electrostatic interactions with heterodimer-stabilizing hydrogen bonds. Herein, EU numbering is used to denote the residues of an immunoglobulin domain when discussing specific modifications that enhance the formation of an antibody (Edelman et al., Proc Natl Acad Sci U S A., 1969, 63: 78-85).

In some embodiments, one or more of the CH3 domains of the first polypeptide chain and one or more of the CH3 domains of the second polypeptide chain of the second antibody are associated via a KiH modification.

In some embodiments, the CH3 domain of the first polypeptide chain and the CH3 domain of the second polypeptide chain of the second antibody each comprise a KiH modification.

Any suitable KiH modifications known in the art may be employed in the practice of the present invention. For example, the T366W ("knob") modification and the T366S, L368A and Y407V ("hole") modifications may be used.

In an embodiment, the first polypeptide chain comprises the T366W modification within the CH3 domain. In an embodiment, the second polypeptide chain comprises the T366S, L368A and Y407V modifications within the CH3 domain. In an embodiment, the first polypeptide chain comprises the T366W modification within the CH3 domain and the second polypeptide chain comprises the T366S, L368A and Y407V modifications within the CH3 domain. In an embodiment, these modifications are according to EU residue numbering (Edelman et al., Proc Natl Acad Sci U S A., 1969, 63: 78-85).

An illustrative CH3 domain comprising the T366W ("knob") modification (denoted in underline) is provided below:

GQPREPQVYTLPPSREEMTKNQVSLWCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 22)

An illustrative CH3 domain comprising the T366S, L368A and Y407V ("hole") modifications (each denoted in underline) is provided below:

GQPREPQVYTLPPSREEMTKNQVSLSCAVKGFYPSDIAVEWESNGQPENNYKTTPPVLDSDG
SFFLVSKLTVDKSRWQQGNVFSCSVMHEALHNHYTQKSLSLSPGK (SEQ ID NO: 23)

In an embodiment, the first polypeptide chain of the second antibody comprises a sequence as set forth in SEQ ID NO: 22, or a sequence having 80%, 85%, 90%, 95%, 96%, 97%, 98%, 99% or 100% identity thereto. In an embodiment, the second polypeptide chain of the second antibody comprises a sequence as set forth in SEQ ID NO: 23, or a sequence having 80%, 85%, 90%, 95%, 96%, 97%, 98%, 99% or 100% identity thereto. In an embodiment, the first polypeptide chain of the second antibody comprises a sequence as set forth in SEQ ID NO: 22, or a sequence having 80%, 85%, 90%, 95%, 96%, 97%, 98%, 99% or 100% identity thereto and the second polypeptide chain of the second antibody comprises a sequence as set forth in SEQ ID NO: 23, or a sequence having 80%, 85%, 90%, 95%, 96%, 97%, 98%, 99% or 100% identity thereto. In a more specific embodiment, the CH3 domains of the first and/or second polypeptide chains comprise or consist of the respective sequences as defined above.

In some embodiments, the at least one inter-chain disulphide bond between the first and second polypeptide chains of the second antibody is formed between a CH3 domain of the first polypeptide chain and a CH3 domain of the second polypeptide chain of the second antibody.

In an embodiment, a CH3 domain of the first polypeptide chain forms a disulphide bond with a CH3 domain of the second polypeptide chain. In a preferred embodiment, a CH3 domain of the first polypeptide chain forms a disulphide bond with a CH3 domain of the second polypeptide chain, and in addition one or more of the CH3 domains of the first polypeptide chain and/or one or more of the CH3 domains of the second polypeptide chain comprise a modification that enhances the affinity between the first and second polypeptide chains.

In some embodiments, at least one inter-chain disulphide bond between the first and second polypeptide chains of the second antibody is an asymmetric disulphide bond.

In some embodiments, one or more of the CH3 domains of the first polypeptide chain and one or more of the CH3 domains of the second polypeptide chain of the second antibody comprise a modification to introduce a cysteine residue to promote the formation of a disulphide bond between the CH3 domains of the first and second polypeptide chains.

In some embodiments, the modification(s) to introduce a cysteine residue to promote the formation of a disulphide bond between the CH3 domains of the first and second polypeptide chains are configured to provide an asymmetric disulphide bond.

Suitably, the S354C mutation and the Y349C mutation described herein may be used to provide an asymmetric disulphide bond between the CH3 domains of the first and second polypeptide chains.

Accordingly, in one embodiment, the first polypeptide chain comprises the S354C modification within the CH3 domain. In an embodiment, the second polypeptide chain comprises the Y349C modification within the CH3 domain. In an embodiment of the second antibody, the first polypeptide chain comprises the S354C modification within the CH3 domain and the second polypeptide chain comprises the Y349C modification within the CH3 domain.

An illustrative CH3 domain comprising the S354C modification (denoted in underline) is provided below:

GQPREPQVYTLPPCREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 24)

An illustrative CH3 domain comprising the Y349C modification (denoted in underline) is provided below:

GQPREPQVCTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 25)

In an embodiment, the first polypeptide chain of the second antibody comprises a sequence as set forth in SEQ ID NO: 24, or a sequence having 80%, 85%, 90%, 95%, 96%, 97%, 98%, 99% or 100% identity thereto. In an embodiment, the second polypeptide chain of the second antibody comprises a sequence as set forth in SEQ ID NO: 25, or a sequence having 80%, 85%, 90%, 95%, 96%, 97%, 98%, 99% or 100% identity thereto. In an embodiment, the first polypeptide chain of the second antibody comprises a sequence as set forth in SEQ ID NO: 24, or a sequence having 80%, 85%, 90%, 95%, 96%, 97%, 98%, 99% or 100% identity thereto and the second polypeptide chain of the second antibody comprises a sequence as set forth in SEQ ID NO: 25, or a sequence having 80%, 85%, 90%, 95%, 96%, 97%, 98%, 99% or 100% identity thereto. In a more specific embodiment, the CH3 domains of the first and/or second polypeptide chains of the second antibody comprise or consist of the respective sequences as defined above.

In a preferred embodiment, the modification that promotes the dimerisation of the first and second polypeptide chains (e.g. the KiH modification) is compatible with the modification to provide a disulphide bond (e.g. an asymmetric disulphide bond) between the CH3 domains of the first and second polypeptide chains as described herein.

In some embodiments, one or more of the CH3 domains of the first polypeptide chain of the second antibody and one or more of the CH3 domains of the second polypeptide chain of the second antibody are associated via a KiH modification and one or more of the CH3 domains of the first polypeptide chain of the second antibody and one or more of the CH3 domains of the second polypeptide chain of the second antibody comprise a modification to introduce a cysteine residue to promote the formation of a disulphide bond between the CH3 domains of the first and second polypeptide chains of the second antibody. In an embodiment, the modifications to introduce a cysteine residue to promote the formation of a disulphide bond between the CH3 domains of the first and second polypeptide chains of the second antibody are configured to provide an asymmetric disulphide bond.

In some embodiments, the CH3 domain of the first polypeptide chain of the second antibody and the CH3 domain of the second polypeptide chain of the second antibody each comprise a KiH modification and a modification to introduce a cysteine residue to promote the formation of a disulphide bond between the CH3 domains of the first and second polypeptide chains of the second antibody. In an embodiment, the modifications to introduce a cysteine residue to promote the formation of a disulphide bond between the CH3 domains of the first and second polypeptide chains of the second antibody are configured to provide an asymmetric disulphide bond.

In an embodiment, the first polypeptide chain of the second antibody comprises the S354C and T366W modifications within the CH3 domain. In an embodiment, the second polypeptide chain of the second antibody comprises the Y349C, T366S, L368A and Y407V modifications within the CH3 domain. In an embodiment, the first polypeptide chain of the second antibody comprises the S354C and T366W modifications within the CH3 domain and the second polypeptide chain of the second antibody comprises the Y349C, T366S, L368A and Y407V modifications within the CH3 domain. In an embodiment, these modifications are according to EU residue numbering.

An illustrative CH3 domain comprising the S354C modification and T366W ("knob") modification (each denoted in underline) is provided below:

GQPREPQVYTLPPCREEMTKNQVSLWCLVKGFYPSDIAVEWESNGQPENNYKTTPPVLDSDG
SFFLYSKLTVDKSRWQQGNVFSCSVMHEALHNHYTQKSLSLSPGK (SEQ ID NO: 2)

An illustrative CH3 domain comprising the Y349C modification and the T366S, L368A and Y407V ("hole") modifications (each denoted in underline) is provided below:

GQPREPQVCTLPPSREEMTKNQVSLSCAVKGFYPSDIAVEWESNGQPENNYKTTPPVLDSDG
SFFLVSKLTVDKSRWQQGNVFSCSVMHEALHNHYTQKSLSLSPGK (SEQ ID NO: 3)

In an embodiment, the first polypeptide chain of the second antibody comprises a sequence as set forth in SEQ ID NO: 2, or a sequence having 80%, 85%, 90%, 95%, 96%, 97%, 98%, 99% or 100% identity thereto. In an embodiment, the second polypeptide chain of the second antibody comprises a sequence as set forth in SEQ ID NO: 3, or a sequence having 80%, 85%, 90%, 95%, 96%, 97%, 98%, 99% or 100% identity thereto. In an embodiment, the first polypeptide chain of the second antibody comprises a sequence as set forth in SEQ ID NO: 2, or a sequence having 80%, 85%, 90%, 95%, 96%, 97%, 98%, 99% or 100% identity thereto and the second polypeptide chain of the second antibody comprises a sequence as set forth in SEQ ID NO: 3, or a sequence having 80%, 85%, 90%, 95%, 96%, 97%, 98%, 99% or 100% identity thereto. In a more specific embodiment, the CH3 domains of the first and/or second polypeptide chains comprise or consist of the respective sequences as defined above.

In some embodiments, the first polypeptide chain of the second antibody comprises one or more constant immunoglobulin domains and one or more variable immunoglobulin domains N-terminal of the constant immunoglobulin domains, wherein all of the variable immunoglobulin domains N-terminal of the constant immunoglobulin domains of the first polypeptide chain are single domain variable heavy immunoglobulin (VHH) domains, and wherein the second polypeptide chain of the second antibody comprises one or more constant immunoglobulin domains, wherein all of the constant immunoglobulin domains in the first polypeptide chain and the second polypeptide chain are constant heavy 3 (CH3) domains.

By "N-terminal of the CH3 domain" it is meant that the VHH is located N-terminal of the CH3 domain by any means known in the art. In an embodiment, the VHH is fused directly to the N-terminus of the CH3 domain, i.e. the VHH domain may be immediately adjacent to the CH3 domain. However, in an embodiment, the VHH domain is connected to the N-terminus of the CH3 domain by a linking sequence as described herein.

In some embodiments, the second antibody is a VHH-CH3 fusion.

As used herein, the term "VHH-CH3 fusion" refers to a polypeptide comprising one or more VHH domains fused N-terminal to a CH3 domain. Suitably, the antibody is a dimer comprising two polypeptides, each polypeptide comprising one or more VHH domains fused N-terminal to a CH3 domain.

In a further aspect, the invention provides a co-delivery system comprising one or more nucleic acid molecules encoding a first antibody and a second antibody, wherein each of the first antibody and the second antibody comprises at least a first polypeptide chain and a second polypeptide chain covalently linked by at least one inter-chain disulphide bond, wherein all of the inter-chain disulphide bonds of the first antibody are at different positions to the inter-chain disulphide bonds of the second antibody, wherein the first antibody is an IgG antibody, and wherein the second antibody is a VHH-CH3 fusion.

In some embodiments, the second polypeptide chain of the second antibody further comprises one or more variable immunoglobulin domains N-terminal of the constant immunoglobulin domains, wherein all of the variable immunoglobulin domains N-terminal of the CH3 domains are single domain variable heavy immunoglobulin (VHH) domains.

In some embodiments, the first polypeptide chain and the second polypeptide chain of the second antibody each comprises a single VHH domain N-terminal of the CH3 domains.

In some embodiments, the first polypeptide chain of the second antibody further comprises at least one binding moiety or at least one cognate of a binding moiety C-terminal of the CH3 domains.

By "C-terminal of the CH3 domain" it is meant that the VHH is located C-terminal of the CH3 domain by any means known in the art. In an embodiment, the VHH is fused directly to the C-terminus of the CH3 domain, i.e. the VHH domain may be immediately adjacent to the CH3 domain. However, in an embodiment, the VHH domain is connected to the C-terminus of the CH3 domain by a linking sequence as described herein.

In some embodiments:
a) within the first polypeptide chain of the second antibody the sequence between the CH3 domain(s) and the VHH domain(s) located N-terminal of the CH3 domain(s) consists of a first linking sequence; and
b) within the second polypeptide chain of the second antibody the sequence between the CH3 domain(s) and the VHH domains located N-terminal of the CH3 domains consists of a second linking sequence.

Herein, it will be understood that in an embodiment the VHH domains N-terminal of the CH3 domains in each of the first and second polypeptide chains of the second antibody are fused directly to the respective CH3 domains. Thus, in an embodiment the VHH domains N-terminal of the CH3 domains are immediately adjacent to the CH3 domains. Thus, in an embodiment there is no other polypeptide sequence present between the VHH domains N-terminal of the CH3 domains and the CH3 domains. However, in an alternative embodiment, there is a polypeptide sequence present between the VHH domains N-terminal of the CH3 domains and the CH3 domains. In embodiments, this sequence is referred to as the linking sequence. In an embodiment where a linking sequence is not present, this may also be defined as the linking sequence comprising 0 amino acids.

The linking sequence may comprise one or more linkers (e.g. GS linkers) and/or an antibody hinge region sequence, such as those linkers and antibody hinge regions that are widely known in the art. In an embodiment, each of the linking sequences comprises a GS linker and additional sequences. In an embodiment, each of the linking sequences comprises in the following order a first GS linker, an antibody hinge region, and a second GS linker. In an embodiment, each of the linking sequences consists of a GS linker. In an embodiment, the first linking sequence is not capable of forming a disulphide bond with the second linking sequence when present in the context of the constructs of the present invention.

An illustrative linking sequence, the classical minibody-hinge region (denoted in *italics*), as described in the literature, is provided below:
*DKTHTCPPCGGGSSGGGSG* (SEQ ID NO: 4)

A further illustrative linking sequence, the hinge region of the classical ΔCH2-IgG (denoted in *italics),* as described in the literature, is provided below:
*EPKSCDKTHTCPPCGGGSSGGGSG* (SEQ ID NO: 5)

A further illustrative linking sequence, the full-length G1:G3/PAP hinge sequence (i.e. comprising the G1:G3/PAP hinge sequence (denoted in *italics)* and glycine-serine linker) as described in the literature, is provided below:
*EPKSCDKTHTCPPCPEPKSCDTPPPCPRCPAPGGGSSGGGSG* (SEQ ID NO: 6)

A further illustrative linking sequence, the "truncated G1:G3/PAP hinge sequence" (i.e. comprising a truncated G1 :G3/PAP hinge sequence (denoted in *italics)* preceded by a glycine-serine linker) is provided below:
*GGGGSGGGGSDKTHTCPPCPEPKSCDTPPPCPRCPAPGGGSSGGGSG* (SEQ ID NO: 7)

A further illustrative linking sequence, the "truncated ΔCH2-hinge" (comprising a truncated ΔCH2-hinge sequence (denoted in *italics)* preceded by a glycine-serine linker), is provided below:
*GGGGSGGGGSDKTHTCPPCGGGSSGGGSG* (SEQ ID NO: 8)

As can be seen, the truncated ΔCH2-hinge sequence shown in *italics* in SEQ ID NO: 8 above corresponds to the classical minibody-hinge sequence shown in *italics* in SEQ ID NO: 4 above. Hence, the terms "truncated ΔCH2-hinge", "minibody-hinge" and "minibody-linker" may be used herein to refer to this sequence.

A further illustrative linking sequence, the truncated ΔCH2-hinge comprising cysteine to serine modifications (i.e. the truncated ΔCH2-hinge sequence (denoted in *italics)* containing cysteine to serine modifications (denoted in underline) preceded by a glycine-serine linker), is provided below:
*GGGGSGGGGSDKTHTSPPSGGGSSGGGSG* (SEQ ID NO: 9)

A further illustrative linking sequence, the short glycine-serine linker, is provided below:
GGGGS (SEQ ID NO: 10)

In an embodiment, the linking sequence of the first and/or second polypeptide chain comprises or consists of a sequence as set forth in SEQ ID NO: 4, or a sequence having 80%, 85%, 90%, 95%, 96%, 97%, 98%, 99% or 100% identity thereto.

In an embodiment, the linking sequence of the first and/or second polypeptide chain comprises or consists of a sequence as set forth in SEQ ID NO: 5, or a sequence having 80%, 85%, 90%, 95%, 96%, 97%, 98%, 99% or 100% identity thereto.

In an embodiment, the linking sequence of the first and/or second polypeptide chain comprises or consists of a sequence as set forth in SEQ ID NO: 6, or a sequence having 80%, 85%, 90%, 95%, 96%, 97%, 98%, 99% or 100% identity thereto.

In a preferred embodiment, the linking sequence of the first and/or second polypeptide chain comprises or consists of a sequence as set forth in SEQ ID NO: 7, or a sequence having 80%, 85%, 90%, 95%, 96%, 97%, 98%, 99% or 100% identity thereto.

In a preferred embodiment, the linking sequence of the first and/or second polypeptide chain comprises or consists of a sequence as set forth in SEQ ID NO: 8, or a sequence having 80%, 85%, 90%, 95%, 96%, 97%, 98%, 99% or 100% identity thereto.

In a preferred embodiment, the linking sequence of the first and/or second polypeptide chain comprises or consists of a sequence as set forth in SEQ ID NO: 9, or a sequence having 80%, 85%, 90%, 95%, 96%, 97%, 98%, 99% or 100% identity thereto.

In a preferred embodiment, the linking sequence of the first and/or second polypeptide chain comprises or consists of a sequence as set forth in SEQ ID NO: 10, or a sequence having 80%, 85%, 90%, 95%, 96%, 97%, 98%, 99% or 100% identity thereto.

The first linking sequence and second linking sequence may be the same sequence or different sequences. Preferably, the first and second linking sequences are the same.

The linking sequence may include cysteine residues or be devoid of cysteine residues. Preferably, the linking sequence is devoid of cysteine residues. Thus, the first and second linking sequences within the second antibody may form inter-chain disulphides or may not form any inter-chain disulphide bonds. Preferably, the first and second linking sequences within the second antibody do not form inter-chain disulphide bonds. In a preferred embodiment, the first linking sequence does not form a disulphide bond with the second linking sequence. In particular, the linking sequences may be devoid of any cysteine residues in an embodiment wherein the CH3 domains form a disulphide bond as described herein.

The linking sequence may comprise a hinge region of an immunoglobulin or an antibody fragment. Suitably, the linking sequence may comprise the minibody/ΔCH2-hinge or the G1/G3:PAP-hinge. Suitably, the linking sequence may comprise the truncated ΔCH2-hinge or the truncated G1/G3:PAP-hinge as described herein. Suitably, the linking sequence may comprise or consist of a Glycine-Serine (GS) linker. In an embodiment, the linking sequence does not comprise a hinge region. In some embodiments, each of the first and second linking sequences consists of a Glycine-Serine (GS) linker. Suitable GS linkers for use in accordance with the invention are described elsewhere herein.

In some embodiments, each of the first and second linking sequences comprises as few as or consists of 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0 amino acids.

In some embodiments, each of the first and second linking sequences comprises as few as or consists of 30, 29, 28, 27, 26, 25, 24, 23, 22, 21, 20, 19, 18, 17, 16, 15, 14, 13, 12, 11, 10, 9, 8, 7, 6, 5, 4, 3, 2, 1, 0 amino acids.

In some embodiments, each of the first and second linking sequences consists of 2 to 5 amino acids. Suitably, each of the first and second linking sequences consists of 2 amino acids. Suitably, each of the first and second linking sequences consists of 3 amino acids. Suitably, each of the first and second linking sequences consists of 4 amino acids. Suitably, each of the first and second linking sequences consists of 5 amino acids.

### Illustrative VHH-CH3 fusion sequences

Illustrative sequences of bispecific, trivalent VHH-CH3 fusions for use according to the invention are provided below. The positions of the VHH domains within the antibodies are indicated for illustrative purposes only.

As described herein, in some embodiments, the second antibody, third antibody and/or or any one of the one or more additional antibodies may be a VHH-CH3 fusion.

### Key:

Regular font = CH3 domain
*Italics* = truncated G1:G3/PAP hinge sequence or truncated ΔCH2-hinge sequence
Underline = cysteine to serine modifications
Highlight = sequence of glycine-serine linkers

### 1) Illustrative VHH-CH3 fusion comprising a truncated G1/G3:PAP hinge and KiH modifications in the CH3 domains

### First polypeptide sequence:

[VHH1 sequence]-

GGGGSGGGGSDKTHTCPPCPEPKSCDTPPPCPRCPAPGGGSSGGGSGGQPREPQVYTLPPCR
EEMTKNQVSLWCLVKGFYPSDIAVEWESNGQPENNYKTTPPVLDSDGSFFLYSKLTVDKSRW
QQGNVFSCSVMHEALHNHYTQKSLSLSPGKGGGGSGGGS-[VHH2 sequence] (SEQ
ID NO: 11)

### Second polypeptide sequence:

[VHH3-Sequence]-

GGGGSGGGGSDKTHTCPPCPEPKSCDTPPPCPRCPAPGGGSSGGGSGGQPREPQVCTLPPSR
EEMTKNQVSLSCAVKGFYPSDIAVEWESNGQPENNYKTTPPVLDSDGSFFLVSKLTVDKSRW
QQGNVFSCSVMHEALHNHYTQKSLSLSPGK (SEQ ID NO: 12)

### 2) VHH-CH3 fusion comprising a truncated ΔCH2-hinge and KiH modifications in the CH3 domains

### First polypeptide sequence:

[VHH1 sequence]-

GGGGSGGGGSDKTHTCPPCGGGSSGGGSGGQPREPQVYTLPPCREEMTKNQVSLWCLVKGFY
PSDIAVEWESNGQPENNYKTTPPVLDSDGSFFLYSKLTVDKSRWQQGNVFSCSVMHEALHNH
YTQKSLSLSPGKGGGGSGGGS-[VHH2 sequence] (SEQ ID NO: 13)

### Second polypeptide sequence:

*[VHH3 sequence]-*

GGGGSGGGGSDKTHTCPPCGGGSSGGGSGGQPREPQVCTLPPSREEMTKNQVSLSCAVKGFY
PSDIAVEWESNGQPENNYKTTPPVLDSDGSFFLVSKLTVDKSRWQQGNVFSCSVMHEALHNH
YTQKSLSLSPGK (SEQ ID NO: 14)

### 3) VHH-CH3 fusion comprising a truncated ΔCH2-hinge containing cysteine to serine modifications and KiH modifications in the CH3 domains

### First polypeptide sequence:

[VHH1 sequence]-

GGGGSGGGGSDKTHTSPPSGGGSSGGGSGGQPREPQVYTLPPCREEMTKNQVSLWCLVKGFY
PSDIAVEWESNGQPENNYKTTPPVLDSDGSFFLYSKLTVDKSRWQQGNVFSCSVMHEALHNH
YTQKSLSLSPGKGGGGSGGGS-[VHH2 sequence] (SEQ ID NO: 15)

### Second polypeptide sequence:

[VHH3 sequence]-

GGGGSGGGGSDKTHTSPPSGGGSSGGGSGGQPREPQVCTLPPSREEMTKNQVSLSCAVKGFY
PSDIAVEWESNGQPENNYKTTPPVLDSDGSFFLVSKLTVDKSRWQQGNVFSCSVMHEALHNH
YTQKSLSLSPGK (SEQ ID NO: 16)

### 4) VHH-CH3 fusion comprising a short glycine-serine linker and KiH modifications in the CH3 domains

### First polypeptide sequence:

[VHH1 sequence]-

GGGGSGQPREPQVYTLPPCREEMTKNQVSLWCLVKGFYPSDIAVEWESNGQPENNYKTTPPV
LDSDGSFFLYSKLTVDKSRWQQGNVFSCSVMHEALHNHYTQKSLSLSPGKGGGGSGGGS-
[VHH2 sequence] (SEQ ID NO: 17)

### Second polypeptide sequence:

[VHH3 sequence]-

GGGGSGQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK (SEQ ID NO:
18)

In one embodiment, the first polypeptide chain comprises a sequence as set forth in SEQ ID NO: 11, or a sequence having 80%, 85%, 90%, 95%, 96%, 97%, 98%, 99% or 100% identity thereto.

In one embodiment, the first polypeptide chain comprises a sequence as set forth in SEQ ID NO: 13, or a sequence having 80%, 85%, 90%, 95%, 96%, 97%, 98%, 99% or 100% identity thereto.

In one embodiment, the first polypeptide chain comprises a sequence as set forth in SEQ ID NO: 15, or a sequence having 80%, 85%, 90%, 95%, 96%, 97%, 98%, 99% or 100% identity thereto.

In one embodiment, the first polypeptide chain comprises a sequence as set forth in SEQ ID NO: 17, or a sequence having 80%, 85%, 90%, 95%, 96%, 97%, 98%, 99% or 100% identity thereto.

In one embodiment, the second polypeptide chain comprises a sequence as set forth in SEQ ID NO: 12, or a sequence having 80%, 85%, 90%, 95%, 96%, 97%, 98%, 99% or 100% identity thereto.

In one embodiment, the second polypeptide chain comprises a sequence as set forth in SEQ ID NO: 14, or a sequence having 80%, 85%, 90%, 95%, 96%, 97%, 98%, 99% or 100% identity thereto.

In one embodiment, the second polypeptide chain comprises a sequence as set forth in SEQ ID NO: 16, or a sequence having 80%, 85%, 90%, 95%, 96%, 97%, 98%, 99% or 100% identity thereto.

In one embodiment, the second polypeptide chain comprises a sequence as set forth in SEQ ID NO: 18, or a sequence having 80%, 85%, 90%, 95%, 96%, 97%, 98%, 99% or 100% identity thereto.

In one embodiment, the first polypeptide chain comprises a sequence as set forth in SEQ ID NO: 11, or a sequence having 80%, 85%, 90%, 95%, 96%, 97%, 98%, 99% or 100% identity thereto and the second polypeptide chain comprises a sequence as set forth in SEQ ID NO: 12, or a sequence having 80%, 85%, 90%, 95%, 96%, 97%, 98%, 99% or 100% identity thereto.

In one embodiment, the first polypeptide chain comprises a sequence as set forth in SEQ ID NO: 13, or a sequence having 80%, 85%, 90%, 95%, 96%, 97%, 98%, 99% or 100% identity thereto and the second polypeptide chain comprises a sequence as set forth in SEQ ID NO: 14, or a sequence having 80%, 85%, 90%, 95%, 96%, 97%, 98%, 99% or 100% identity thereto.

In one embodiment, the first polypeptide chain comprises a sequence as set forth in SEQ ID NO: 15, or a sequence having 80%, 85%, 90%, 95%, 96%, 97%, 98%, 99% or 100% identity thereto and the second polypeptide chain comprises a sequence as set forth in SEQ ID NO: 16, or a sequence having 80%, 85%, 90%, 95%, 96%, 97%, 98%, 99% or 100% identity thereto.

In one embodiment, the first polypeptide chain comprises a sequence as set forth in SEQ ID NO: 17, or a sequence having 80%, 85%, 90%, 95%, 96%, 97%, 98%, 99% or 100% identity thereto and the second polypeptide chain comprises a sequence as set forth in SEQ ID NO: 18, or a sequence having 80%, 85%, 90%, 95%, 96%, 97%, 98%, 99% or 100% identity thereto.

### Illustrative IgG sequences

Illustrative sequences of bispecific, tetravalent IgG antibodies for use according to the invention are provided below. The positions of the VHH domains within the antibodies are indicated for illustrative purposes only.

As described herein, in some embodiments, the first antibody, third antibody and/or or any one of the one or more additional antibodies may be an IgG antibody or an antibody derived from an IgG antibody (e.g. an IgG-like antibody).

### Illustrative IgG heavy chain sequence - VH-CH1-Hinge-CH2-CH3-VHH:

[VH sequence]-

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGKGGGGSGGGS-[VHH2 sequence] (SEQ ID NO: 19)

### Illustrative IgG light chain sequence - VL-CL (kappa):

[VL sequence]-


 (SEQ ID NO: 20)

### Illustrative IgG light chain sequence - VL-CL (lambda):

[VL sequence]-

GQPKANPTVTLFPPSSEELQANKATLVCLISDFYPGAVTVAWKADSSPVKAGVETTTPSKQS
NNKYAASSYLSLTPEQWKSHRSYSCQVTHEGSTVEKTVAPTECS (SEQ ID NO: 21)

In one embodiment, the first polypeptide chain (e.g. of the first antibody) comprises a sequence as set forth in SEQ ID NO: 19, or a sequence having 80%, 85%, 90%, 95%, 96%, 97%, 98%, 99% or 100% identity thereto.

In one embodiment, the second polypeptide chain (e.g. of the first antibody) comprises a sequence as set forth in SEQ ID NO: 20, or a sequence having 80%, 85%, 90%, 95%, 96%, 97%, 98%, 99% or 100% identity thereto.

In one embodiment, the second polypeptide chain (e.g. of the first antibody) comprises a sequence as set forth in SEQ ID NO: 21, or a sequence having 80%, 85%, 90%, 95%, 96%, 97%, 98%, 99% or 100% identity thereto.

In one embodiment, the first polypeptide chain (e.g. of the first antibody) comprises a sequence as set forth in SEQ ID NO: 19, or a sequence having 80%, 85%, 90%, 95%, 96%, 97%, 98%, 99% or 100% identity thereto and the second polypeptide chain (e.g. of the first antibody) comprises a sequence as set forth in SEQ ID NO: 20, or a sequence having 80%, 85%, 90%, 95%, 96%, 97%, 98%, 99% or 100% identity thereto.

In one embodiment, the first polypeptide chain (e.g. of the first antibody) comprises a sequence as set forth in SEQ ID NO: 19, or a sequence having 80%, 85%, 90%, 95%, 96%, 97%, 98%, 99% or 100% identity thereto and the second polypeptide chain (e.g. of the first antibody) comprises a sequence as set forth in SEQ ID NO: 21, or a sequence having 80%, 85%, 90%, 95%, 96%, 97%, 98%, 99% or 100% identity thereto.

### Additional antibodies

In some embodiments, the one or more nucleic acid molecules further encodes one or more additional antibodies, preferably one, two, three, four or five additional antibodies.

In one embodiment, the one or more nucleic acid molecules further encodes an additional antibody. In one embodiment, the one or more nucleic acid molecules further encodes two additional antibodies. In one embodiment, the one or more nucleic acid molecules further encodes three additional antibodies. In one embodiment, the one or more nucleic acid molecules further encodes four additional antibodies. In one embodiment, the one or more nucleic acid molecules further encodes five additional antibodies.

In some embodiments, each additional antibody is independently selected from the group consisting of a human antibody, a humanized antibody, a chimeric antibody, a multispecific antibody, a monoclonal antibody, a polyclonal antibody, or any combination thereof.

In some embodiments, any one of the one or more of the additional antibodies is a monoclonal antibody.

In some embodiments, each additional antibody is a monoclonal antibody.

In some embodiments, any one of the one or more of the additional antibodies is a multispecific antibody.

In some embodiments, each additional antibody is a multispecific antibody.

In some embodiments, any one of the one or more of the additional antibodies is a monoclonal, multispecific antibody.

In some embodiments, each additional antibody is a monoclonal, multispecific antibody.

In one embodiment, the first polypeptide chain and second polypeptide chain of any one of the one or more additional antibodies are covalently linked by at least two, at least three, at least four, at least five, at least six, at least seven, or at least eight inter-chain disulphide bonds.

In one embodiment, the first polypeptide chain and second polypeptide chain of each of the one or more additional antibodies are covalently linked by at least two, at least three, at least four, at least five, at least six, at least seven, or at least eight inter-chain disulphide bonds.

In one embodiment, the first polypeptide chain and second polypeptide chain of any one of the one or more additional antibodies are covalently linked by one, two, three, four, five, six, seven, or eight inter-chain disulphide bonds.

In one embodiment, the first polypeptide chain and second polypeptide chain of each of the one or more additional antibodies are covalently linked by one, two, three, four, five, six, seven, or eight inter-chain disulphide bonds.

In one embodiment, the first polypeptide chain and second polypeptide chain of any one of the one or more additional antibodies are covalently linked by one inter-chain disulphide bond. In one embodiment, the first polypeptide chain and second polypeptide chain of each of the one or more additional antibodies are covalently linked by one inter-chain disulphide bond.

In one embodiment, the first polypeptide chain and second polypeptide chain of any one of the one or more additional antibodies are covalently linked by two inter-chain disulphide bonds.

In one embodiment, the first polypeptide chain and second polypeptide chain of each of the one or more additional antibodies are covalently linked by two inter-chain disulphide bonds.

In one embodiment, any one of the one or more additional antibodies further comprises a third polypeptide chain and a fourth polypeptide chain.

In one embodiment, each of the one or more additional antibodies further comprises a third polypeptide chain and a fourth polypeptide chain.

In one embodiment, the third polypeptide chain is covalently linked by an inter-chain disulphide bond to the first polypeptide chain of the antibody.

In one embodiment, the fourth polypeptide chain is covalently linked by an inter-chain disulphide bond to the second polypeptide chain of the antibody.

In one embodiment, any one of the one or more additional antibodies is an IgG antibody.

In one embodiment, each of the one or more additional antibodies is an IgG antibody.

In one embodiment, any one of the one or more additional antibodies is a VHH-CH3 fusion.

In one embodiment, each of the one or more additional antibodies is a VHH-CH3 fusion.

In one embodiment, the first polypeptide chain of any one of the one or more additional antibodies comprises at least one binding moiety or at least one cognate of a binding moiety at the N- and/or C-terminus.

In one embodiment, the second polypeptide chain of any one of the one or more additional antibodies comprises at least one binding moiety or at least one cognate of a binding moiety at the N- and/or C-terminus.

In one embodiment the first polypeptide chain of any one of the one or more additional antibodies comprises at least one binding moiety or at least one cognate of a binding moiety at the N- and/or C-terminus and the second polypeptide chain of said one or more additional antibody comprises at least one binding moiety or at least one cognate of a binding moiety at the N- and/or C-terminus.

In one embodiment, the first polypeptide chain of any one of the one or more additional antibodies comprises at least one binding moiety at the N-terminus.

In one embodiment, the first polypeptide chain of any one of the one or more additional antibodies comprises at least one binding moiety at the C-terminus.

In one embodiment, the first polypeptide chain of any one of the one or more additional antibodies comprises at least one binding moiety at the N-terminus and at least one binding moiety at the C-terminus.

In one embodiment, the first polypeptide chain of any one of the one or more additional antibodies comprises at least one cognate of a binding moiety at the N-terminus.

In one embodiment, the first polypeptide chain of any one of the one or more additional antibodies comprises at least one cognate of a binding moiety at the C-terminus.

In one embodiment, the first polypeptide chain of any one of the one or more additional antibodies comprises at least one cognate of a binding moiety at the N-terminus and at least one cognate of a binding moiety at the C-terminus.

In one embodiment, the first polypeptide chain of any one of the one or more additional antibodies comprises at least one binding moiety at the N-terminus and at least one cognate of a binding moiety at the C-terminus.

In one embodiment, the first polypeptide chain of any one of the one or more additional antibodies comprises at least one binding moiety at the C-terminus and at least one cognate of a binding moiety at the N-terminus.

In one embodiment, the second polypeptide chain of any one of the one or more additional antibodies comprises at least one binding moiety at the N-terminus.

In one embodiment, the second polypeptide chain of any one of the one or more additional antibodies comprises at least one binding moiety at the C-terminus.

In one embodiment, the second polypeptide chain of any one of the one or more additional antibodies comprises at least one binding moiety at the N-terminus and at least one binding moiety at the C-terminus.

In one embodiment, the second polypeptide chain of any one of the one or more additional antibodies comprises at least one cognate of a binding moiety at the N-terminus.

In one embodiment, the second polypeptide chain of any one of the one or more additional antibodies comprises at least one cognate of a binding moiety at the C-terminus.

In one embodiment, the second polypeptide chain of any one of the one or more additional antibodies comprises at least one cognate of a binding moiety at the N-terminus and at least one cognate of a binding moiety at the C-terminus.

In one embodiment, the second polypeptide chain of any one of the one or more additional antibodies comprises at least one binding moiety at the N-terminus and at least one cognate of a binding moiety at the C-terminus.

In one embodiment, the second polypeptide chain of any one of the one or more additional antibodies comprises at least one binding moiety at the C-terminus and at least one cognate of a binding moiety at the N-terminus.

Suitably, the binding moiety is as described herein.

Suitably, the cognate of a binding moiety is as described herein.

In some embodiments, each of the one or more additional antibodies comprises at least a first polypeptide chain and a second polypeptide chain, wherein the first polypeptide chain and the second polypeptide chain are covalently linked by at least one inter-chain disulphide bond, and wherein all of the inter-chain disulphide bonds between the first polypeptide chain and the second polypeptide chain of the one or more additional antibodies are at different positions to the inter-chain disulphide bonds between the first polypeptide chain and the second polypeptide chain in each of the first antibody and the second antibody.

In some embodiments, all of the inter-chain disulphide bonds between the first polypeptide chain and the second polypeptide chain of the one or more additional antibodies are outside the hinge region.

In some embodiments, the first polypeptide chain and the second polypeptide chain of any one of the one or more additional antibodies further comprises one or more constant heavy 3 (CH3) domains.

In some embodiments, the first polypeptide chain and the second polypeptide chain of each of the one or more additional antibodies each further comprises one or more constant heavy 3 (CH3) domains.

In one embodiment, the first polypeptide chain and the second polypeptide chain of each of the additional antibodies comprise a single CH3 domain.

In one embodiment, any one of the one or more additional antibodies comprises a modification that enhances the formation of the antibody.

In one embodiment, each of the one or more additional antibodies comprises a modification that enhances the formation of the antibody.

Suitably, each of the additional antibodies comprises an asymmetric disulphide bond as described herein.

In some embodiments, one or more of the CH3 domains of the first polypeptide chain and/or one or more of the CH3 domains of the second polypeptide chain of any one of the one or more additional antibodies comprise a modification that enhances the formation of the antibody.

In some embodiments, one or more of the CH3 domains of the first polypeptide chain and/or one or more of the CH3 domains of the second polypeptide chain of each of the one or more additional antibodies comprise a modification that enhances the formation of the antibody.

In some embodiments, the modification that enhances the formation of the antibody is a 'knobs-into-holes' modification.

Suitably, the modification that enhances the formation of the antibody is as described herein.

In some embodiments, at least one inter-chain disulphide bond of each of the one or more additional antibodies is an asymmetric disulphide bond as described herein.

Suitably, any one of the additional antibodies comprises an asymmetric disulphide bond as described herein.

In some embodiments, one or more of the CH3 domains of the first polypeptide chain and/or one or more of the CH3 domains of the second polypeptide chain of the second antibody and of each of the one or more additional antibodies comprise different modifications that enhance the formation of the antibody.

By "different modifications that enhance the formation of the antibody" it is meant that different types of modifications are used (e.g. KiH modifications and modifications that promote electrostatic interactions) or that modifications of the same type but at different residue locations or comprising different specific mutations are used.

In one embodiment, the one or more nucleotides encode one additional antibody, wherein one or more of the CH3 domains of the first polypeptide chain and one or more of the CH3 domains of the second polypeptide chain of the second antibody are associated via a 'knobs-into-holes' modification and one or more of the CH3 domains of the first polypeptide and one or more of the CH3 domains of the second polypeptide of the additional antibody comprise a modification that promotes electrostatic interactions between the first and second polypeptide chains of the additional antibody.

In some embodiments, one or more of the CH3 domains of the first polypeptide chain and/or one or more of the CH3 domains of the second polypeptide chain of the second antibody and of each of the one or more additional antibodies comprise the same modification that enhances the formation of the antibody.

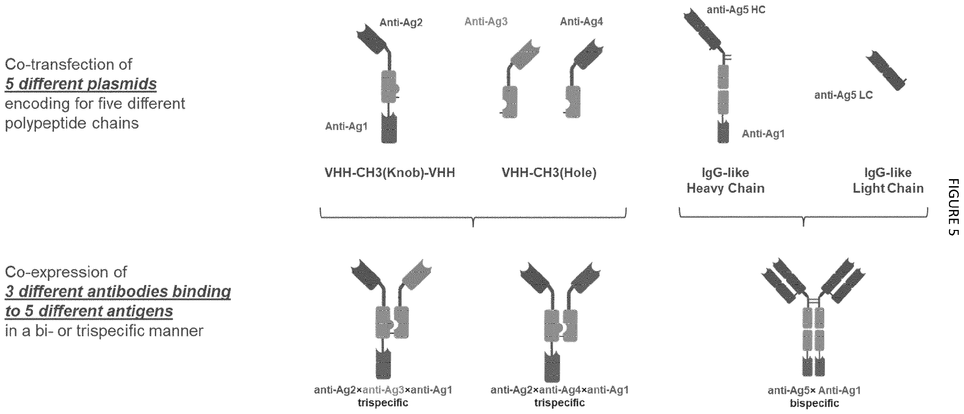

By "the same modification that enhances the formation of the antibody" it is meant that the identical modification is used. For example, identical KiH modifications may be employed. This may be desirable where the first polypeptide chain of the second antibody and of the one or more additional antibodies is identical (see, for example, Figure 5).

In some embodiments, each of the one or more additional antibodies comprises at least a first polypeptide chain and a second polypeptide chain, wherein the first polypeptide chain and the second polypeptide chain are covalently linked by at least one inter-chain disulphide bond, wherein the first polypeptide chain and the second polypeptide chain of each of the one or more additional antibodies each further comprises one or more constant heavy 3 (CH3) domains, wherein one or more of the CH3 domains of the first polypeptide chain and/or one or more of the CH3 domains of the second polypeptide chain of each of the one or more additional antibodies comprises a modification that enhances the formation of the antibody, and wherein the first polypeptide chain of the second antibody and the first polypeptide chain of each of the one or more additional antibodies is identical.

In some embodiments, the second polypeptide chain of the second antibody is different to the second polypeptide chain of each of the one or more additional antibodies.

Suitably, since the first polypeptide chain of the second antibody and of each of the one or more additional antibodies is identical, the first polypeptide chain and the second polypeptide chain of the second antibody are covalently linked by at least one inter-chain disulphide bond that is at the same position as the at least one inter-chain disulphide bond linking the first polypeptide chain and the second polypeptide chain of each of the one or more additional antibodies.

### Third antibody

In some embodiments, the one or more nucleic acid molecules further encode(s) a third antibody comprising a first polypeptide chain and a second polypeptide chain covalently linked by at least one inter-chain disulphide bond.

In some embodiments, the third antibody is a monoclonal antibody.

In some embodiments, the third antibody is a multispecific antibody.

In some embodiments, the third antibody is a monoclonal, multispecific antibody.

In one embodiment, the first polypeptide chain and second polypeptide chain of the third antibody are covalently linked by at least two, at least three, at least four, at least five, at least six, at least seven, or at least eight inter-chain disulphide bonds.

In one embodiment, the first polypeptide chain and second polypeptide chain of the third antibody are covalently linked by one, two, three, four, five, six, seven, or eight inter-chain disulphide bonds.

In one embodiment, the first polypeptide chain and second polypeptide chain of the third antibody are covalently linked by two inter-chain disulphide bonds.

In one embodiment, the third antibody further comprises a third polypeptide chain and a fourth polypeptide chain.

In one embodiment, the third polypeptide chain is covalently linked by an inter-chain disulphide bond to the first polypeptide chain of the antibody.

In one embodiment, the fourth polypeptide chain is covalently linked by an inter-chain disulphide bond to the second polypeptide chain of the antibody.

In one embodiment, the third antibody is an IgG antibody.

In one embodiment, the third antibody is a VHH-CH3 fusion.

In some embodiments, the first polypeptide chain of the third antibody comprises at least one binding moiety or at least one cognate of a binding moiety at the N- and/or C-terminus.

In some embodiments, the second polypeptide chain of the third antibody comprises at least one binding moiety or at least one cognate of a binding moiety at the N- and/or C-terminus.

In some embodiments, the first polypeptide chain of the third antibody comprises at least one binding moiety or at least one cognate of a binding moiety at the N- and/or C-terminus and the second polypeptide chain of the third antibody comprises at least one binding moiety or at least one cognate of a binding moiety at the N- and/or C-terminus.

In one embodiment, the first polypeptide chain of the third antibody comprises at least one binding moiety at the N-terminus.

In one embodiment, the first polypeptide chain of the third antibody comprises at least one binding moiety at the C-terminus.

In one embodiment, the first polypeptide chain of the third antibody comprises at least one binding moiety at the N-terminus and at least one binding moiety at the C-terminus.

In one embodiment, the first polypeptide chain of the third antibody comprises at least one cognate of a binding moiety at the N-terminus.

In one embodiment, the first polypeptide chain of the third antibody comprises at least one cognate of a binding moiety at the C-terminus.

In one embodiment, the first polypeptide chain of the third antibody comprises at least one cognate of a binding moiety at the N-terminus and at least one cognate of a binding moiety at the C-terminus.

In one embodiment, the first polypeptide chain of the third antibody comprises at least one binding moiety at the N-terminus and at least one cognate of a binding moiety at the C-terminus.

In one embodiment, the first polypeptide chain of the third antibody comprises at least one binding moiety at the C-terminus and at least one cognate of a binding moiety at the N-terminus.

In one embodiment, the second polypeptide chain of the third antibody comprises at least one binding moiety at the N-terminus.

In one embodiment, the second polypeptide chain of the third antibody comprises at least one binding moiety at the C-terminus.

In one embodiment, the second polypeptide chain of the third antibody comprises at least one binding moiety at the N-terminus and at least one binding moiety at the C-terminus.

In one embodiment, the second polypeptide chain of the third antibody comprises at least one cognate of a binding moiety at the N-terminus.

In one embodiment, the second polypeptide chain of the third antibody comprises at least one cognate of a binding moiety at the C-terminus.

In one embodiment, the second polypeptide chain of the third antibody comprises at least one cognate of a binding moiety at the N-terminus and at least one cognate of a binding moiety at the C-terminus.

In one embodiment, the second polypeptide chain of the third antibody comprises at least one binding moiety at the N-terminus and at least one cognate of a binding moiety at the C-terminus.

In one embodiment, the second polypeptide chain of the third antibody comprises at least one binding moiety at the C-terminus and at least one cognate of a binding moiety at the N-terminus.

Suitably, the binding moiety is as described herein.

Suitably, the cognate of a binding moiety is as described herein.

In some embodiments, the third antibody comprises at least a first polypeptide chain and a second polypeptide chain, wherein the first polypeptide chain and the second polypeptide chain are covalently linked by at least one inter-chain disulphide bond, and wherein all of the inter-chain disulphide bonds between the first polypeptide chain and the second polypeptide chain of the third antibody are at different positions to the inter-chain disulphide bonds between the first polypeptide chain and the second polypeptide chain in each of the first antibody and the second antibody.

In some embodiments, all of the inter-chain disulphide bonds between the first polypeptide chain and the second polypeptide chain of the third antibody are outside the hinge region.

In some embodiments, the co-delivery system further comprises a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond formed between a CH3 domain of the first polypeptide and a CH3 domain of the second polypeptide, and wherein all of the inter-chain disulphide bonds between the first polypeptide chain and the second polypeptide chain of the third antibody are outside the hinge region.

In some embodiments, the first polypeptide chain and the second polypeptide chain of the third antibody further comprises one or more constant heavy 3 (CH3) domains.

In one embodiment, the first polypeptide chain and the second polypeptide chain of the third antibody comprise a single CH3 domain.

In one embodiment, the third antibody comprises a modification that enhances the formation of the antibody.

In some embodiments, one or more of the CH3 domains of the first polypeptide chain and/or one or more of the CH3 domains of the second polypeptide chain of the third antibody comprise a modification that enhances the formation of the antibody.

In some embodiments, the modification that enhances the formation of the antibody is a 'knobs-into-holes' modification.

Suitably, the modification that enhances the formation of the antibody is as described herein.

Suitably, the third antibody comprises an asymmetric disulphide bond as described herein.

In some embodiments, at least one inter-chain disulphide bond of the third antibody is an asymmetric disulphide bond as described herein.

In some embodiments, one or more of the CH3 domains of the first polypeptide chain and/or one or more of the CH3 domains of the second polypeptide chain of the second antibody and of the third antibody comprise different modifications that enhance the formation of the antibody.

In some embodiments, the third antibody comprises at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein all of the inter-chain disulphide bonds between the first polypeptide chain and the second polypeptide chain of the third antibody are at different positions to the inter-chain disulphide bonds between the first polypeptide chain and the second polypeptide chain in each of the first antibody and the second antibody, and wherein one or more of the CH3 domains of the first polypeptide and one or more of the CH3 domains of the second polypeptide of the third antibody comprise a modification that promotes electrostatic interactions between the first and second polypeptide chains of the third antibody.

Suitably, the modification that promotes electrostatic interactions between the first and second polypeptide chains of the third antibody may be as described herein. For example, the modification may be electrostatic steering employing DD-KK variants with asymmetric electrostatic interactions (Gunasekaran et al., J Biol Chem., 2010 , 285: 19637-46): DEKK dimerisation technology (https://merus.nl/technology/multiclonics-platform/ and Nardis et al, J Biol Chem., 2017, 292: 14706-14717): HA-TF variants with asymmetric hydrophobic interactions (Moore et al., MAbs, 2011, 3: 546-57): EW-RVT variants, which were designed to replace the conserved electrostatic interactions with asymmetric hydrophobic interactions and to add asymmetric long-range electrostatic interactions at the rim of the heterodimeric CH3 interface (Choi et al., Mol Cancer Ther., 2013, 12: 2748-59): and the K370E_{CH3A}-E357N_{CH3B} mutations employed in the "A107" variant which replaced the homodimer-favouring electrostatic interactions with heterodimer-stabilizing hydrogen bonds. Herein, EU numbering is used to denote the residues of an immunoglobulin domain when discussing specific modifications that enhance the formation of an antibody (Edelman et al., Proc Natl Acad Sci U S A., 1969, 63: 78-85).

Suitably, one or more of the CH3 domains of the first polypeptide chain and one or more of the CH3 domains of the second polypeptide chain of the second antibody are associated via a 'knobs-into-holes' modification.

In some embodiments, the co-delivery system further comprises a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein the first polypeptide chain and the second polypeptide chain of the third antibody each further comprises one or more constant heavy 3 (CH3) domains, wherein one or more of the CH3 domains of the first polypeptide chain and/or one or more of the CH3 domains of the second polypeptide chain of the third antibody comprises a modification that enhances the formation of the antibody, and wherein the first polypeptide chain of the second antibody and the first polypeptide chain of the third antibody is identical.

In some embodiments, the second polypeptide chain of the second antibody is different to the second polypeptide chain of the third antibody.

In some embodiments, one or more of the CH3 domains of the first polypeptide chain and/or one or more of the CH3 domains of the second polypeptide chain of the second antibody and of the third antibody comprise the same modification that enhances the formation of the antibody.

By "the same modification that enhances the formation of the antibody" it is meant that the identical modification is used. For example, identical KiH modifications may be employed. This may be desirable where the first polypeptide chain of the second antibody and of the third antibody is identical (see, for example, Figure 5). Suitably, since the first polypeptide chain of the second antibody and of the third antibody is identical, the first polypeptide chain and the second polypeptide chain of the second antibody are covalently linked by at least one inter-chain disulphide bond that is at the same position as the at least one inter-chain disulphide bond linking the first polypeptide chain and the second polypeptide chain of the third antibody.

In some embodiments, when at least one inter-chain disulphide bond of the second antibody is an asymmetric disulphide bond, the asymmetric disulphide bond of the second antibody is different to the asymmetric disulphide bond of the third antibody.

By different asymmetric disulphide bonds it is meant that the disulphide bonds are located at different residue positions within the polypeptide chains of the second and third antibodies. The residue positions may be determined as described herein, e.g. using the Kabat, EU or IMGT numbering systems.

In some embodiments, one or more of the CH3 domains of the first polypeptide chain and one or more of the CH3 domains of the second polypeptide chain of the third antibody are associated via a 'knobs-into-holes' modification.

Suitably, when one or more of the CH3 domains of the first polypeptide chain and one or more of the CH3 domains of the second polypeptide chain of the second antibody are associated via a 'knobs-into-holes' modification, the third antibody comprises a different 'knobs-into-holes' modification or all of the disulphide bond(s) between the first and second polypeptide chains of the third antibody which is at a different position to all of the disulphide bond(s) between the first and second polypeptide chains of the second antibody. In this way, mispairing of the chains of the second and third antibodies is avoided.

### Vector

In one aspect, the invention provides a vector comprising the co-delivery system of the invention.

In an embodiment, the one or more nucleic acid sequences described herein are in the form of a vector. In an embodiment, the present invention provides a vector comprising the one or more nucleic acid sequences described herein. Such a vector may be used to introduce the nucleic acid sequence(s) into a host cell so that it expresses the antibodies as described herein.

In an embodiment, the vector comprises a plurality of nucleic acid sequences which encode different component of the antibodies as provided by the present invention. For example, in an embodiment the vector comprises a first nucleic acid sequence which encodes the first polypeptide of the first antibody and a second nucleic acid sequence which encodes the second polypeptide of the first antibody, as well as a third nucleic acid sequence which encodes the first polypeptide of the second antibody.

In embodiments, the vector may, for example, be a plasmid or a viral vector, such as a retroviral vector or a lentiviral vector, or a transposon based vector or synthetic mRNA.

In an embodiment, the vector is capable of transfecting or transducing a cell.

### Particles

The co-delivery system of the present invention or the vector of the present invention may be present in particles comprising (i) the one or more nucleic acid sequences as described herein and (ii) at least one cationic or cationically ionizable compound such as a polymer or lipid complexing the one or more nucleic acid sequences. Electrostatic interactions between positively charged molecules such as polymers and lipids and negatively charged nucleic acid are involved in particle formation. This results in complexation and spontaneous formation of nucleic acid particles comprising the one or more nucleic acid sequences.

Accordingly, in a further aspect, the invention provides a nucleic acid particle comprising the co-delivery system of the invention. In a further aspect, the invention provides a nucleic acid particle comprising the vector of the invention.

Different types of nucleic acid containing particles have been described previously to be suitable for delivery of nucleic acid (e.g. RNA) in particulate form (cf., *e.g.,* Kaczmarek, J. C. et al., 2017, Genome Medicine 9, 60). For non-viral delivery vehicles, nanoparticle encapsulation of nucleic acids physically protects nucleic acids from degradation and, depending on the specific chemistry, can aid in cellular uptake and endosomal escape.

In the context of the present disclosure, the term "particle" relates to a structured entity formed by molecules or molecule complexes, in particular particle forming compounds. In some embodiments, the particle contains an envelope (*e.g.,* one or more layers or lamellas) made of one or more types of amphiphilic substances (*e.g.,* amphiphilic lipids). In this context, the expression "amphiphilic substance" means that the substance possesses both hydrophilic and lipophilic properties. The envelope may also comprise additional substances (*e.g.,* additional lipids) which do not have to be amphiphilic. Thus, the particle may be a monolamellar or multilamellar structure, wherein the substances constituting the one or more layers or lamellas comprise one or more types of amphiphilic substances (in particular selected from the group consisting of amphiphilic lipids) optionally in combination with additional substances (*e.g.,* additional lipids) which do not have to be amphiphilic. In some embodiments, the term "particle" relates to a micro- or nano-sized structure, such as a micro- or nano-sized compact structure. According to the present disclosure, the term "particle" includes nanoparticles.

A "nucleic acid particle" can be used to deliver nucleic acid sequences to a target site of interest (*e.g*., cell, tissue, organ, and the like). A nucleic acid particle may be formed from lipids comprising at least one cationic or cationically ionizable lipid or lipid-like material. Without intending to be bound by any theory, it is believed that the cationic or cationically ionizable lipid or lipid-like material combines together with the nucleic acids to form aggregates, and this aggregation results in colloidally stable particles.

Nucleic acid particles (such as RNA particles and/or DNA particles) include lipid nanoparticle (LNP)-based and lipoplex (LPX)-based formulations.

In general, a lipoplex (LPX) is obtainable from mixing two aqueous phases, namely a phase comprising nucleic acid (such as RNA and/or DNA) and a phase comprising a dispersion of lipids. In some embodiments, the lipid phase comprises liposomes.

In some embodiments, liposomes are self-closed unilamellar or multilamellar vesicular particles wherein the lamellae comprise lipid bilayers and the encapsulated lumen comprises an aqueous phase. A prerequisite for using liposomes for nanoparticle formation is that the lipids in the mixture as required are able to form lamellar (bilayer) phases in the applied aqueous environment.

In some embodiments, liposomes comprise unilamellar or multilamellar phospholipid bilayers enclosing an aqueous core (also referred to herein as an aqueous lumen). They may be prepared from materials possessing polar head (hydrophilic) groups and nonpolar tail (hydrophobic) groups. In some embodiments, cationic lipids employed in formulating liposomes designed for the delivery of nucleic acids are amphiphilic in nature and consist of a positively charged (cationic) amine head group linked to a hydrocarbon chain or cholesterol derivative via glycerol.

In some embodiments, lipoplexes are multilamellar liposome-based formulations that form upon electrostatic interaction of cationic liposomes with nucleic acids (such as RNAs and/or DNAs). In some embodiments, formed lipoplexes possess distinct internal arrangements of molecules that arise due to the transformation from liposomal structure into compact nucleic acid-lipoplexes (such as RNA- and/or DNA-lipoplexes). In some embodiments, these formulations are characterized by their poor encapsulation of the nucleic acid (such as RNA) and incomplete entrapment of the nucleic acid (such as RNA).

In some embodiments, an LPX particle comprises an amphiphilic lipid, in particular cationic or cationically ionizable amphiphilic lipid, and nucleic acid (such as RNA and/or DNA, especially mRNA) as described herein. In some embodiments, electrostatic interactions between positively charged liposomes (made from one or more amphiphilic lipids, in particular cationic or cationically ionizable amphiphilic lipids) and negatively charged nucleic acid (especially mRNA) results in complexation and spontaneous formation of nucleic acid lipoplex particles. Positively charged liposomes may be generally synthesized using a cationic or cationically ionizable amphiphilic lipid, such as DOTMA and/or DODMA, and additional lipids, such as DOPE. In some embodiments, a nucleic acid (such as RNA and/or DNA, especially mRNA) lipoplex particle is a nanoparticle.

In a further aspect, the invention provides a lipoplex (LPX) comprising the co-delivery system of the invention.

In a further aspect, the invention provides a lipoplex (LPX) comprising the vector of the invention.

In a further aspect, the invention provides a lipid nanoparticle (LNP) comprising the co-delivery system of the invention.

In a further aspect, the invention provides a lipid nanoparticle (LNP) comprising the vector of the invention.

In general, a lipid nanoparticle (LNP) is obtainable from direct mixing of nucleic acid (such as RNA and/or DNA) in an aqueous phase with lipids in a phase comprising an organic solvent, such as ethanol. In that case, lipids or lipid mixtures can be used for particle formation, which do not form lamellar (bilayer) phases in water.

In some embodiments, LNPs comprise or consist of a cationic/ionisable lipid and helper lipids such as phospholipids, cholesterol, and/or polyethylene glycol (PEG) lipids. In some embodiments, in the nucleic acid LNPs (such as RNA and/or DNA LNPs, *e.g*., mRNA LNPs) described herein, the nucleic acid (such as mRNA) is bound by ionisable lipid that occupies the central core of the LNP. In some embodiments, PEG lipid forms the surface of the LNP, along with phospholipids. In some embodiments, the surface comprises a bilayer. In some embodiments, cholesterol and ionisable lipid in charged and uncharged forms can be distributed throughout the LNP.

In some embodiments, nucleic acid (such as RNA and/or DNA, *e.g*., mRNA) may be noncovalently associated with a particle as described herein. In embodiments, the nucleic acid (such as RNA and/or DNA, especially mRNA) may be adhered to the outer surface of the particle (surface nucleic acid) and/or may be contained in the particle (encapsulated nucleic acid (such as encapsulated DNA).

In some embodiments, the particles (e.g., LNPs and LPXs) described herein have a size (such as a diameter) in the range of about 10 to about 2000 nm, such as at least about 15 nm (e.g., at least about 20 nm, at least about 25 nm, at least about 30 nm, at least about 35 nm, at least about 40 nm, at least about 45 nm, at least about 50 nm, at least about 55 nm, at least about 60 nm, at least about 65 nm, at least about 70 nm, at least about 75 nm, at least about 80 nm, at least about 85 nm, at least about 90 nm, at least about 95 nm, or at least about 100 nm) and/or at most 1900 nm (e.g., at most about 1900 nm, at most about 1800 nm, at most about 1700 nm, at most about 1600 nm, at most about 1500 nm, at most about 1400 nm, at most about 1300 nm, at most about 1200 nm, at most about 1100 nm, at most about 1000 nm, at most about 950 nm, at most about 900 nm, at most about 850 nm, at most about 800 nm, at most about 750 nm, at most about 700 nm, at most about 650 nm, at most about 600 nm, at most about 550 nm, or at most about 500 nm), such as in the range of about 20 to about 1500 nm, such as about 30 to about 1200 nm, about 40 to about 1100 nm, about 50 to about 1000 nm, about 60 to about 900 nm, about 70 to 800 nm, about 80 to 700 nm, about 90 to 600 nm, or about 50 to 500 nm or about 100 to 500 nm, such as in the range of 10 to 1000 nm, 15 to 500 nm, 20 to 450 nm, 25 to 400 nm, 30 to 350 nm, 40 to 300 nm, 50 to 250 nm, 60 to 200 nm, or 70 to 150 nm.

In some embodiments, the particles described herein are nanoparticles. The term "nanoparticle" relates to a nano-sized particle comprising nucleic acid (such as RNA and/or DNA, e.g., mRNA) as described herein and at least one cationic or cationically ionisable lipid, wherein all three external dimensions of the particle are in the nanoscale, *i.e.,* at least about 1 nm and below about 1000 nm. Preferably, the size of a particle is its diameter.

### Composition

In a further aspect, the invention provides a composition comprising the co-delivery system, vector, or nucleic acid particle of the invention.

The composition may be a pharmaceutical composition.

The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

### Therapeutic use

In a further aspect, the invention provides the co-delivery system, vector, nucleic acid particle, or composition of the invention for use in a method of therapy.

In a further aspect, the invention provides the co-delivery system, vector, nucleic acid particle or composition of the invention for use in a diagnostic method.

In a further aspect, the invention provides the use of the co-delivery system, vector, nucleic acid particle, or composition of the invention for the manufacture of a medicament.

In a further aspect, the invention provides the use of the co-delivery system, vector, nucleic acid particle, or composition of the invention for the manufacture of a diagnostic agent.

In a further aspect, the invention provides a method of therapy comprising administering the co-delivery system, vector, nucleic acid particle, or composition of the invention to a subject.

In a further aspect, the invention provides a diagnostic method comprising administering the co-delivery system, vector, nucleic acid particle, or composition of the invention to a subject.

A method for therapy includes a method for treating a disease as well as a method for preventing a disease.

A method for treating a disease relates to the therapeutic use of the co-delivery system, vector, nucleic acid particle, or composition according to the invention. In this respect, the co-delivery system, vector, nucleic acid particle, or composition according to the invention may be administered to a subject having an existing disease or condition in order to lessen, reduce or improve at least one symptom associated with the disease and/or to slow down, reduce or block the progression of the disease.

The method for preventing a disease relates to the prophylactic use of the co-delivery system, vector, nucleic acid particle, or composition according to the invention. In this respect, the co-delivery system, vector, nucleic acid particle, or composition according to the invention may be administered to a subject who has not yet contracted the disease and/or who is not showing any symptoms of the disease to prevent or impair the cause of the disease or to reduce or prevent development of at least one symptom associated with the disease. The subject may have a predisposition for, or be thought to be at risk of developing, the disease.

Suitably, the vector(s) or nucleic acids may be introduced by transduction. Suitably, the vector(s) or nucleic acids may be introduced by transfection.

The disease or condition to be treated and/or prevented may be cancer, an immune-related disorder, an autoimmune disease, a metabolic disease, an infectious disease, a cardiovascular disease, an inflammatory disease.

Suitably, the cancer may be a solid tumour or a liquid tumour.

The cancer may be a cancer such as neuroblastoma, prostate cancer, bladder cancer, breast cancer, colon cancer, endometrial cancer, kidney cancer (renal cell), leukaemia, lung cancer, melanoma, non-Hodgkin lymphoma, pancreatic cancer, and thyroid cancer.

Suitably, the disease or condition may be asthma, arthritis, psoriasis, Crohn's disease, transplant rejection, migraine, headaches.

### General definitions

It is to be understood herein that references to any feature in respect of the 'first polypeptide "and/or" the second polypeptide', or the 'first polypeptide "and, where present," the second polypeptide' have the same meaning, and include independent selections of a) the feature in question applying to the first polypeptide alone; b) the feature in question applying to the second polypeptide alone, and c) the feature in question applying to both the first and second polypeptides. For example, a statement of "wherein the first polypeptide comprises a single VHH domain and the second polypeptide, where present, comprises a single VHH domain", refers to embodiments wherein explicitly a) the first polypeptide comprises a single VHH domain, b) the second polypeptide comprises a single VHH domain and c) the first and second polypeptides each comprise a single VHH domain. It will be understood that each of these embodiments may apply to the constructs and polypeptides of the present invention as appropriate.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino (N) to carboxy (C) orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

The term "polypeptide" is used in the conventional sense to mean a series of amino acids, typically L-amino acids, connected one to the other, typically by peptide bonds between the α-amino and carboxyl groups of adjacent amino acids. The term "polypeptide" is used interchangeably with the terms "amino acid sequence", "peptide" and/or "protein". The term "residues" is used to refer to amino acids in an amino acid sequence.

The term "variant" in relation to a polypeptide refers to a polypeptide that has an equivalent function to the amino acid sequences described herein, but which includes one or more amino acid substitutions, insertions or deletions.

As used herein, the terms "polynucleotide", "nucleotide", "nucleic acid sequence" and "nucleic acid" are intended to be synonymous with each other.

The terms "variant", "homologue" or "derivative" in relation to a nucleotide sequence include any substitution of, variation of, modification of, replacement of, deletion of or addition of one (or more) nucleic acid from or to the sequence.

"Sequence identity" between two nucleic acid sequences indicates the percentage of nucleotides that are identical between the sequences. The terms "% identical" and "% identity" or similar terms are intended to refer, in particular, to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the global homology algorithm by Needleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (e.g., at blast.ncbi.nlm.nih.gov/Blast.cgi?PAGE_TYPE=BlastSearch&BLAST_SPEC=blast2seq&LINK _LOC=align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared (e.g., the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence.

In some embodiments, "isolated" means removed (e.g., purified) from the natural state or from an artificial composition, such as a composition from a production process. For example, a nucleic acid, peptide or polypeptide naturally present in a living animal is not "isolated", but the same nucleic acid, peptide or polypeptide partially or completely separated from the coexisting materials of its natural state is "isolated". An isolated nucleic acid, peptide or polypeptide can exist in substantially purified form, or can exist in a non-native environment such as, for example, a host cell.

The term "expression" as used herein is defined as the transcription and/or translation of a particular nucleotide sequence.

In the context of the present disclosure, the term "transcription" relates to a process, wherein the genetic code in a DNA sequence is transcribed into RNA (especially mRNA). Subsequently, the RNA may be translated into peptide or polypeptide.

With respect to RNA, the term "expression" or "translation" relates to the process in the ribosomes of a cell by which a strand of mRNA directs the assembly of a sequence of amino acids to make a peptide or polypeptide.

The term "nucleic acid" comprises deoxyribonucleic acid (DNA), ribonucleic acid (RNA), combinations thereof, and modified forms thereof. The term comprises genomic DNA, cDNA, mRNA, recombinantly produced and chemically synthesized molecules. A nucleic acid may be present as a single-stranded or double-stranded and linear or covalently circularly closed molecule. A nucleic acid can be isolated. The term "isolated nucleic acid" means, according to the present disclosure, that the nucleic acid (i) was amplified in vitro, for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "nucleoside" (abbreviated herein as "N") relates to compounds which can be thought of as nucleotides without a phosphate group. While a nucleoside is a nucleobase linked to a sugar (e.g., ribose or deoxyribose), a nucleotide is composed of a nucleoside and one or more phosphate groups. Examples of nucleosides include cytidine, uridine, pseudouridine, adenosine, and guanosine.

The five standard nucleosides which usually make up naturally occurring nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

A modified purine (A or G) or pyrimidine (C, T, or U) base moiety is preferably modified by one or more alkyl groups, more preferably one or more C1-4 alkyl groups, even more preferably one or more methyl groups. Particular examples of modified purine or pyrimidine base moieties include N7-alkyl-guanine, N6-alkyl-adenine, 5-alkyl-cytosine, 5-alkyl-uracil, and N(1)-alkyl-uracil, such as N7-C1-4 alkyl-guanine, N6-C1-4 alkyl-adenine, 5-C1-4 alkyl-cytosine, 5-C1-4 alkyl-uracil, and N(1)-C1-4 alkyl-uracil, preferably N7-methyl-guanine, N6-methyl-adenine, 5-methyl-cytosine, 5-methyl-uracil, N1-methyl-pseudouridine, and N(1)-methyl-uracil.

Herein, the term "DNA" relates to a nucleic acid molecule which includes deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

The term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered/modified nucleotides can be referred to as analogs of naturally occurring nucleotides, and the corresponding RNAs containing such altered/modified nucleotides (i.e., altered/modified RNAs) can be referred to as analogs of naturally occurring RNAs. A molecule contains "a majority of ribonucleotide residues" if the content of ribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

"RNA" includes mRNA, tRNA, ribosomal RNA (rRNA), small nuclear RNA (snRNA), self-amplifying RNA (saRNA), single-stranded RNA (ssRNA), dsRNA, inhibitory RNA (such as antisense ssRNA, small interfering RNA (siRNA), or microRNA (miRNA)), activating RNA (such as small activating RNA) and immunostimulatory RNA (isRNA). In some embodiments, "RNA" refers to mRNA.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of" also include the term "consisting of'.

### Embodiments of the invention

Various preferred features and embodiments of the present invention will now be described with reference to the following numbered paragraphs:
1. A co-delivery system comprising one or more nucleic acid molecules encoding a first antibody and a second antibody,
   wherein each of the first antibody and the second antibody comprises at least a first polypeptide chain and a second polypeptide chain covalently linked by at least one inter-chain disulphide bond, and
   wherein all of the inter-chain disulphide bonds between the first polypeptide chain and the second polypeptide chain of the first antibody are at different positions to the inter-chain disulphide bonds between the first polypeptide chain and the second polypeptide chain of the second antibody.
2. The co-delivery system according to paragraph 1, wherein all of the at least one inter-chain disulphide bonds between the first polypeptide chain and the second polypeptide chain of the first antibody are within the hinge region.
3. The co-delivery system according to paragraph 1 or paragraph 2, wherein all of the inter-chain disulphide bonds between the first polypeptide chain and the second polypeptide chain of the second antibody are outside the hinge region.
4. A co-delivery system comprising one or more nucleic acid molecules encoding a first antibody and a second antibody,
   wherein each of the first antibody and the second antibody comprises at least a first polypeptide chain and a second polypeptide chain,
   wherein the first polypeptide chain and the second polypeptide chain of the first antibody are covalently linked by at least one inter-chain disulphide bond within the hinge region,
   wherein the first polypeptide chain and the second polypeptide chain of the second antibody are covalently linked by at least one inter-chain disulphide bond, and
   wherein all of the inter-chain disulphide bonds between the first polypeptide chain and the second polypeptide chain of the second antibody are outside the hinge region.
5. The co-delivery system according to any one of the preceding paragraphs, wherein each nucleic acid molecule encodes at least one polypeptide chain of an antibody.
6. The co-delivery system according to any one of the preceding paragraphs, wherein the nucleic acid molecule(s) is DNA and/or RNA.
7. The co-delivery system according to any one of the preceding paragraphs, wherein the first polypeptide chain of the first antibody comprises at least one binding moiety or at least one cognate of a binding moiety at the N- and/or C-terminus.
8. The co-delivery system according to any one of the preceding paragraphs, wherein the first polypeptide chain of the second antibody comprises at least one binding moiety or at least one cognate of a binding moiety at the N- and/or C-terminus.
9. The co-delivery system according to any one of the preceding paragraphs, wherein the second polypeptide chain of the first antibody comprises at least one binding moiety or at least one cognate of a binding moiety at the N- and/or C-terminus.
10. The co-delivery system according to any one of the preceding paragraphs, wherein the second polypeptide chain of the second antibody comprises at least one binding moiety or at least one cognate of a binding moiety at the N- and/or C-terminus.
11. The co-delivery system according to any one of paragraphs 7 to 10, wherein the binding moiety is selected from the list consisting of a single-chain variable fragment (scFv); a Fab; a Fab'; a F(ab)'2; an Fv; a single domain antibody (sdAb); a VHH; a single chain variable domain; a designed ankyrin repeat protein (DARPin); and an aptamer.
12. The co-delivery system according to any one of the preceding paragraphs, wherein the first and second antibodies are independently selected from the group consisting of a human antibody, a humanized antibody, a chimeric antibody, a multispecific antibody, a monoclonal antibody, a polyclonal antibody, or any combination thereof.
13. The co-delivery system according to any one of the preceding paragraphs, wherein the first and/or second antibody is a multispecific antibody.
14. The co-delivery system according to any one of the preceding paragraphs, wherein each of the first and second antibodies is a multispecific antibody.
15. The co-delivery system according to any one of the preceding paragraphs, wherein the one or more nucleic acid molecules further encodes one or more additional antibodies, preferably one, two, three, four or five additional antibodies.
16. The co-delivery system according to any one of the preceding paragraphs, wherein the first antibody comprises an Fc region.
17. The co-delivery system according to any one of the preceding paragraphs, wherein the first antibody is an IgG antibody.
18. The co-delivery system according to any one of the preceding paragraphs, wherein the first polypeptide chain and the second polypeptide chain of the second antibody each further comprises one or more constant heavy 3 (CH3) domains.
19. The co-delivery system according to any one of the preceding paragraphs, wherein the second antibody is a VHH-CH3 fusion.
20. A co-delivery system comprising one or more nucleic acid molecules encoding a first antibody and a second antibody,
   wherein each of the first antibody and the second antibody comprises at least a first polypeptide chain and a second polypeptide chain covalently linked by at least one inter-chain disulphide bond,
   wherein all of the inter-chain disulphide bonds of the first antibody are at different positions to the inter-chain disulphide bonds of the second antibody,
   wherein the first antibody is an IgG antibody, and
   wherein the second antibody is a VHH-CH3 fusion.
21. The co-delivery system according to any one of paragraphs 18 to 20, wherein the first polypeptide chain and the second polypeptide chain of the second antibody each comprises a single CH3 domain.
22. The co-delivery system according to any one of paragraphs 18 to 21, wherein one or more of the CH3 domains of the first polypeptide chain and/or one or more of the CH3 domains of the second polypeptide chain of the second antibody comprise a modification that enhances the formation of the antibody.
23. The co-delivery system according to any one of paragraphs 18 to 22, wherein one or more of the CH3 domains of the first polypeptide chain and/or one or more of the CH3 domains of the second polypeptide chain of the second antibody comprise a modification that enhances the affinity between the first and second polypeptide chains.
24. The co-delivery system according to any one of paragraphs 18 to 23, wherein one or more of the CH3 domains of the first polypeptide chain and/or one or more of the CH3 domains of the second polypeptide chain of the second antibody comprise a modification that reduces steric hindrance to the formation of the antibody.
25. The co-delivery system according to any one of paragraphs 18 to 24, wherein one or more of the CH3 domains of the first polypeptide chain and one or more of the CH3 domains of the second polypeptide chain of the second antibody are associated via a 'knobs-into-holes' modification.
26. The co-delivery system according to any one of paragraphs 18 to 25, wherein the second antibody is a heterodimer.
27. The co-delivery system according to any one of paragraphs 18 to 26, wherein at least one inter-chain disulphide bond is formed between a CH3 domain of the first polypeptide chain and a CH3 domain of the second polypeptide chain of the second antibody.
28. The co-delivery system according to paragraph 27, wherein at least one inter-chain disulphide bond of the second antibody is an asymmetric disulphide bond.
29. The co-delivery system according to paragraph 28, wherein one or more of the CH3 domains of the first polypeptide chain and one or more of the CH3 domains of the second polypeptide chain of the second antibody comprise a modification to introduce a cysteine residue to promote the formation of a disulphide bond between the CH3 domains of the first and second polypeptide chains.
30. The co-delivery system according to paragraph 29, wherein the modification(s) to introduce a cysteine residue to promote the formation of a disulphide bond between the CH3 domains of the first and second polypeptide chains are configured to provide an asymmetric disulphide bond.
31. The co-delivery system according to any one of paragraphs 19 to 30, wherein the first polypeptide chain of the second antibody comprises one or more constant immunoglobulin domains and one or more variable immunoglobulin domains N-terminal of the constant immunoglobulin domains, wherein all of the variable immunoglobulin domains N-terminal of the constant immunoglobulin domains of the first polypeptide chain are single domain variable heavy immunoglobulin (VHH) domains, and wherein the second polypeptide chain of the second antibody comprises one or more constant immunoglobulin domains, wherein all of the constant immunoglobulin domains in the first polypeptide chain and the second polypeptide chain are constant heavy 3 (CH3) domains.
32. The co-delivery system according to paragraph 31, wherein the second polypeptide chain of the second antibody further comprises one or more variable immunoglobulin domains N-terminal of the constant immunoglobulin domains, wherein all of the variable immunoglobulin domains N-terminal of the CH3 domains are single domain variable heavy immunoglobulin (VHH) domains.
33. The co-delivery system according to paragraph 31 or paragraph 32, wherein the first polypeptide chain and the second polypeptide chain of the second antibody each comprises a single VHH domain N-terminal of the CH3 domains.
34. The co-delivery system according to any one of paragraphs 31 to 33, wherein the first polypeptide chain of the second antibody further comprises at least one binding moiety or at least one cognate of a binding moiety C-terminal of the CH3 domains.
35. The co-delivery system according to any one of paragraphs 31 to 34, wherein:
   a) within the first polypeptide chain of the second antibody the sequence between the CH3 domain(s) and the VHH domain(s) located N-terminal of the CH3 domain(s) consists of a first linking sequence; and
   b) within the second polypeptide chain of the second antibody the sequence between the CH3 domain(s) and the VHH domains located N-terminal of the CH3 domains consists of a second linking sequence.
36. The co-delivery system according to any one of paragraphs 15 to 35 wherein each of the one or more additional antibodies comprises at least a first polypeptide chain and a second polypeptide chain, wherein the first polypeptide chain and the second polypeptide chain are covalently linked by at least one inter-chain disulphide bond, and wherein all of the inter-chain disulphide bonds between the first polypeptide chain and the second polypeptide chain of the one or more additional antibodies are at different positions to the inter-chain disulphide bonds between the first polypeptide chain and the second polypeptide chain in each of the first antibody and the second antibody.
37. The co-delivery system according to paragraph 36, wherein all of the inter-chain disulphide bonds between the first polypeptide chain and the second polypeptide chain of the one or more additional antibodies are outside the hinge region.
38. The co-delivery system according to paragraph 36 or paragraph 37, wherein the first polypeptide chain and the second polypeptide chain of each of the one or more additional antibodies each further comprises one or more constant heavy 3 (CH3) domains.
39. The co-delivery system according to paragraph 38, wherein one or more of the CH3 domains of the first polypeptide chain and/or one or more of the CH3 domains of the second polypeptide chain of each of the one or more additional antibodies comprise a modification that enhances the formation of the antibody.
40. The co-delivery system according to paragraph 39, wherein one or more of the CH3 domains of the first polypeptide chain and/or one or more of the CH3 domains of the second polypeptide chain of the second antibody and of each of the one or more additional antibodies comprise different modifications that enhance the formation of the antibody.
41. The co-delivery system according to paragraph 40, wherein one or more of the CH3 domains of the first polypeptide chain and/or one or more of the CH3 domains of the second polypeptide chain of the second antibody and of each of the one or more additional antibodies comprise the same modification that enhances the formation of the antibody.
42. The co-delivery system according to any one of paragraphs 22 to 35, wherein each of the one or more additional antibodies comprises at least a first polypeptide chain and a second polypeptide chain, wherein the first polypeptide chain and the second polypeptide chain are covalently linked by at least one inter-chain disulphide bond, wherein the first polypeptide chain and the second polypeptide chain of each of the one or more additional antibodies each further comprises one or more constant heavy 3 (CH3) domains, wherein one or more of the CH3 domains of the first polypeptide chain and/or one or more of the CH3 domains of the second polypeptide chain of each of the one or more additional antibodies comprises a modification that enhances the formation of the antibody, and wherein the first polypeptide chain of the second antibody and the first polypeptide chain of each of the one or more additional antibodies is identical.
43. The co-delivery system according to paragraph 42, wherein the second polypeptide chain of the second antibody is different to the second polypeptide chain of each of the one or more additional antibodies.
44. The co-delivery system according to any one of paragraphs 39 to 43, wherein one or more of the CH3 domains of the first polypeptide and one or more of the CH3 domains of the second polypeptide of each of the one or more additional antibodies are associated via a 'knobs-into-holes' modification.
45. The co-delivery system according to any one of paragraphs 36 to 44, wherein at least one inter-chain disulphide bond of each of the one or more additional antibodies is an asymmetric disulphide bond.
46. The co-delivery system according to any one of paragraphs 25 to 35, wherein the one or more nucleic acid molecules further encodes a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein all of the inter-chain disulphide bonds between the first polypeptide chain and the second polypeptide chain of the third antibody are at different positions to the inter-chain disulphide bonds between the first polypeptide chain and the second polypeptide chain in each of the first antibody and the second antibody, and wherein one or more of the CH3 domains of the first polypeptide and one or more of the CH3 domains of the second polypeptide of the third antibody comprise a modification that promotes electrostatic interactions between the first and second polypeptide chains of the third antibody.
47. The co-delivery system according to paragraph 46, wherein all of the inter-chain disulphide bonds between the first polypeptide chain and the second polypeptide chain of the third antibody are outside the hinge region.
48. The co-delivery system according to any one of paragraphs 22 to 35, wherein the one or more nucleic acid molecules further encodes a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein the first polypeptide chain and the second polypeptide chain of the third antibody each further comprises one or more constant heavy 3 (CH3) domains, wherein one or more of the CH3 domains of the first polypeptide chain and/or one or more of the CH3 domains of the second polypeptide chain of the third antibody comprises a modification that enhances the formation of the antibody, and wherein the first polypeptide chain of the second antibody and the first polypeptide chain of the third antibody is identical.
49. The co-delivery system according to paragraph 48, wherein the second polypeptide chain of the second antibody is different to the second polypeptide chain of the third antibody.
50. The co-delivery system according to any one of paragraphs 1 to 35, wherein the one or more nucleic acid molecules further encodes a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond formed between a CH3 domain of the first polypeptide and a CH3 domain of the second polypeptide, and wherein all of the inter-chain disulphide bonds between the first polypeptide chain and the second polypeptide chain of the third antibody are outside the hinge region.
51. The co-delivery system according to any one of paragraphs 46 to 50, wherein at least one inter-chain disulphide bond of the third antibody is an asymmetric disulphide bond.
52. The co-delivery system according to paragraph 51, wherein, when at least one inter-chain disulphide bond of the second antibody is an asymmetric disulphide bond, the asymmetric disulphide bond of the second antibody is different to the asymmetric disulphide bond of the third antibody.
53. The co-delivery system according to any one of paragraphs 46 to 52, wherein one or more of the CH3 domains of the first polypeptide chain and one or more of the CH3 domains of the second polypeptide chain of the third antibody are associated via a 'knobs-into-holes' modification.
54. A vector comprising the co-delivery system according to any one of the preceding paragraphs.
55. A nucleic acid particle comprising the co-delivery system according to any one of paragraphs 1 to 53 or the vector according to paragraph 54.
56. A composition comprising the co-delivery system according to any one of paragraphs 1 to 53, the vector according to paragraph 54 or the nucleic acid particle according to paragraph 55.
57. The co-delivery system according to any one of paragraphs 1 to 53, the vector according to paragraph 54, the nucleic acid particle according to paragraph 55, or the composition according to paragraph 56 for use in a method of therapy and/or in a diagnostic method.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

### EXAMPLES

### Materials & Methods

### Expression of recombinant fusion proteins using plasmids

Human codon optimized sequences were generated by gene synthesis and cloned into pTWIST expression vector (Twist Bioscience). For recombinant expression of the proteins of interest, Expi293 cells were transfected with ExpiFectamine 293 (ThermoFisher Scientific) using manufacturer's protocol. Culture supernatants were harvested from Expi293 producer cell lines after up to 5 days.

Sequences of the polypeptide chains encoded by the plasmids are detailed below.

### 1) IgG-like antibody

### IgG-like(HC) - VH-CH1-Hinge-CH2-CH3-VHH:

[VH sequence]-

ASTKGPSVFPLAPSSKSTSGGTAALGCLVKDYFPEPVTVSWNSGALTSGVHTFPAVLQSSGL
YSLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTYRVV
SVLTVLHQDWLNGKEYKCKVSNKALPAPIEKTISKAKGQPREPQVYTLPPSREEMTKNQVSL
TCLVKGFYPSDIAVEWESNGQPENNYKTTPPVLDSDGSFFLYSKLTVDKSRWQQGNVFSCSV
MHEALHNHYTQKSLSLSPGKGGGGSGGGS-[VHH2 sequence] (SEQ ID NO: 19)

### IgG-like(LC) - kappa VLCL:

**[VL sequence]-**


 (SEQ ID NO: 20)

### 2) VHH-CH3 fusion comprising a short glycine-serine linker and KiH modifications in the CH3 domains

Regular font denotes the CH3 domain. Underline denotes that KiH modifications (T366W for the knob chain, and T366S, L368A, and Y407V for the hole chain according to the EU numbering scheme). *Underline and italics* denotes the asymmetric disulphide bond modifications (S354C for the knob chain and Y349C for the hole chain according to the EU numbering scheme). Highlight denotes the sequence of glycine-serine linkers.

### VHH-CH3(Knob)-VHH:

[VUH1 sequence]-

GGGGSGQPREPQVYTLPPCREEMTKNQVSLWCLVKGFYPSDIAVEWESNGQPENNYKTTPPV
LDSDGSFFLYSKLTVDKSRWQQGNVFSCSVMHEALHNHYTQKSLSLSPGKGGGGSGGGS-
[VHH2 sequence] (SEQ ID NO: 17)

### VHH-CH3(Hole):

[VHH3 sequence]-

GGGGSGQPREPQVCTLPPSREEMTKNQVSLSCAVKGFYPSDIAVEWESNGQPENNYKTTPPV
LDSDGSFFLVSKLTVDKSRWQQGNVFSCSVMHEALHNHYTQKSLSLSPGK (SEQ ID NO:
18)

### Chain ratio analysis of plasmids

To determine suitable molar plasmid ratios, first the base pairs of the plasmids were set in relative weight ratios to one another. In this step, one chain, most often the light chain-encoding plasmid, was set as "1", meaning that the weight ratios where normalized to this respective plasmid size. Plasmids with more base pairs were consequently calculated to have a normalized weight ratio to the reference plasmid of above 1. Next, suitable molar ratios were chosen between the plasmids. In general, one plasmid was set to a value of 1 where the other plasmids were of lower or higher number of equivalents (e.g. heavy chain plasmid: 1; light chain plasmid 0.8). The steps between the ratios where not greater than 0.25. Based on the normalized weight ratio and the chosen molar ratio, the mass ratio between the plasmids were calculated. These values allowed to calculate, based on the amount of totally transfected plasmids, the amount of each plasmid to apply to the transfection experiment for each chosen molar ratio.

### Western Blot Analysis

For detection of the proteins of interest in the cell culture supernatant, a Western Blot analysis was performed. Cell culture supernatant was mixed with non-reducing Lämmli buffer and subsequently applied to an "Any kD Criterion TGX Stain-Free" gel from Bio-Rad. The SDS-PAGE was loaded with 10-20 µL of a 10-fold diluted supernatant sample and run at 250 V for 40-80 min on ice or at 4°C. Subsequently, the gel was washed in water and transferred onto a nitrocellulose membrane using the Trans-Blot Turbo System of Bio-Rad. The blotted membrane was washed with PBS-T and blocked with 5% milk powder for 1 h at room temperature. After further washing steps and incubation of an anti-VHH-HRP, an anti-kappa-HRP or an anti-human-HRP detection antibody overnight, further wash steps were performed. The HRP substrate was applied, and the Western Blot was analysed using a Bio-Rad ChemiDoc system.

### Jess Western Blot Analysis

For detection of the proteins of interest in the cell culture supernatant, a Western Blot analysis using the Jess device from Bio-Techne was performed. Supernatants were 10-fold diluted and processed as described by the manufacturer. For detection, an anti-VHH-HRP, an anti-kappa-HRP or an anti-human-HRP detection antibody was utilized.

### Binding to multiple antigens

For affinity measurements an Octet HTX device from Sartorius was used.

Commercially available SAX2.0 biosensors were soaked for at least 10 min in kinetic buffer (KB) purchased from the instrument manufacturer. Following a 60 sec baseline in KB, a biotinylated version of one of the antigens was loaded. Subsequently, a quenching step in a 100 µg/mL biocytin solution was performed for 120 sec, followed by a 120 sec baseline in KB. The cell culture supernatant, containing the proteins of interest, was applied to the biosensors until saturation was reached. Subsequently, association of the second antigen was measured for up 1500 sec.

Alternatively, commercially available FAB2G biosensors were soaked for at least 10 min in kinetic buffer (KB) purchased from the instrument manufacturer. Following a 60 sec baseline in KB, the cell culture supernatant was applied, resulting in the immobilization of Fab-containing proteins. This step was performed until 0.7 nm in response was achieved. After subsequent washing steps, soluble antigens or Fc gamma receptors were applied to verify binding.

### Example 1 - Theoretical mispairing and correct pairing of antibody chains during co-delivery

In order to investigate the concept of co-expression of multiple antibodies with no occurrence of mispairing further, the theoretical mispairing combinations were analysed. The inventors assessed the use of two antibody constructs: bispecific, tetravalent IgG-like antibodies in combination with bispecific, trivalent VHH-CH3 fusions (also termed "minibody-like" antibodies).

To facilitate formation of the correct constructs and avoid mispairings, the inventors employed antibody constructs with differential stabilisations of the correctly paired constructs. The IgG-like antibodies employed comprise disulphide bonds within the hinge region to covalently stabilise the correct chain pairings. To create a bispecific, tetravalent antibody, the present inventors fused an additional VHH to the C-terminus of the Fc domain of an IgG antibody to form the IgG-like antibody. The VHH-CH3 fusions analysed comprise KiH modifications of the CH3 domains to promote heterodimer formation as well as an asymmetric disulphide bond between the CH3 domains to covalently stabilise the correct chain pairings. In addition, the VHH-CH3 fusions do not comprise any disulphide bonds within the hinge region, in order to avoid the formation of disulphide bonds with the hinge region of the IgG-like chains. To create a bispecific, trivalent antibody the present inventors fused an additional VHH to the C-terminus of one CH3 domain of the VHH-CH3 fusion.

For co-delivering this VHH-CH3 fusion with the IgG-like antibody, four polypeptide chains need to be simultaneously expressed.

These chains are: VHH-CH3(Knob)-VHH, VHH-CH3(Hole), IgG heavy chain (!gG(HC))-VHH and the IgG light chain (IgG(LC)).

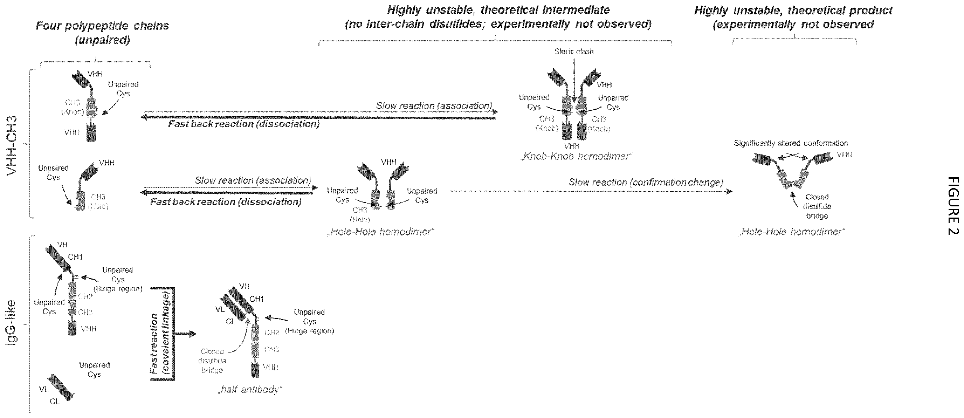

Firstly, the potential intra-construct chain mispairing events were analysed (Figure 2).

For the VHH-CH3 fusions, one intra-construct mispairing species could be a Knob-Knob homodimer. Here, the Knob mutations would lead to a steric clash between both chains making this formation energetically highly unfavourable. The asymmetrical disulphide bridge in the CH3 domains of the VHH-CH3 fusions could not form due to the distance of the respective cysteines. Knob-Knob homodimers are described to occur in literature but are highly unstable and only form under artificial conditions (Kugelstatter *et al.,* 2017).

A further intra-construct mispairing species for the VHH-CH3 fusions could be homodimers of the VHH-CH3 domains containing the Hole mutations. Whilst such Hole-Hole homodimers can occur, these are not stabilized by a disulphide bridge, again due to the distance of the cysteine residues. Upon significant structural alteration of the CH3-CH3 interface, Hole-Hole-homodimers can form and can then even carry a closed disulphide bridge (Kugelstatter et al., 2017). However, this structural alteration mainly happens in the absence of Knob-chains under thermodynamic pressure.

Overall, intra-construct mispairings of the VHH-CH3 fusions were therefore considered by the inventors to be unlikely, especially given the high heterodimer content of classical Fc-based Knobs-into-Holes bispecifics.

For the IgG1-like antibody, intra-construct mispairing can be excluded, as LCs naturally pair with HCs (and this pairing is stabilised with disulphide bonds) and HCs in turn also with HCs. Therefore, the formation of the desired symmetrical !gG1 is unproblematic.

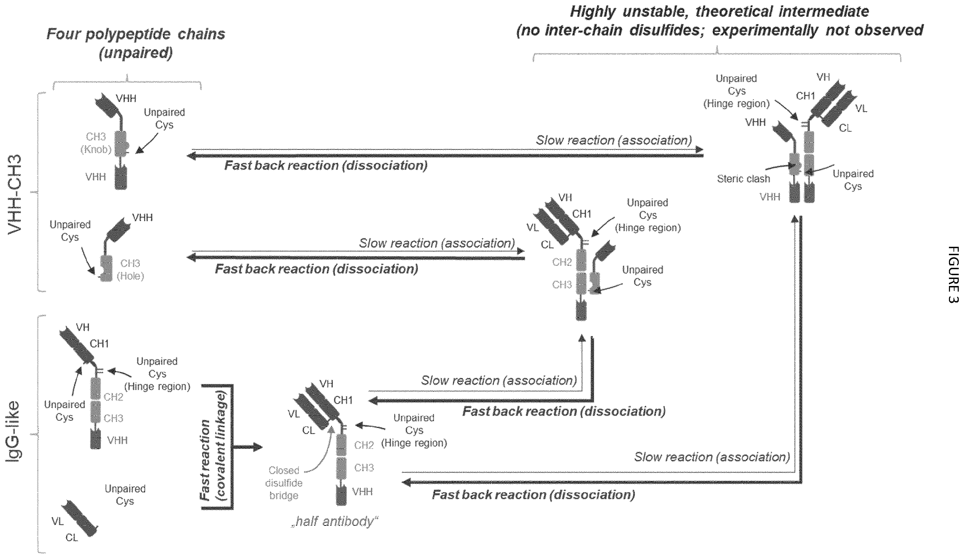

Next, the possibility of inter-construct chain mispairing was analysed (Figure 3).

In naturally occurring antibodies, the Fc is stabilized by the CH3-CH3 interaction as the CH2 domains do not directly interact with one another (Chiu *et al.,* 2019). Therefore, inter-construct CH3-CH3 mispairings were assessed.

In case the CH3 domain of the IgG-like antibody interacts with the CH3 domain of the Knob chain of the VHH-CH3 fusion (VHH-CH3(Knob)-VHH), the steric hindrance mediated by the Knob mutation would destabilize this sub-species. The cysteine within the VHH-CH3(Knob)-VHH chain cannot form a disulphide bond with any cysteine within the IgG-like antibody chain to stabilize the mispaired molecule, neither can the two cysteines within the hinge region of the IgG-like antibody form a disulphide bond with any cysteine within the VHH-CH3(Knob)-VHH chain. Hence, as these chains are not covalently attached to one another, they can dissociate easily. The same is true if a transient complex of IgG-like HC and VHH-CH3(Hole) is formed. The observation that CH3 interactions can be transient and dissociate rapidly if another, energetically more favourable pairing option is available, is reported in literature multiple times (Labrijn *et al.,* 2014; Dengle *et al.,* 2020; Dickopf *et al.,* 2022).

Overall, inter-construct mispairings were therefore considered by the inventors to be unlikely, especially given the steric hindrance and easy dissociation of these sub-species.

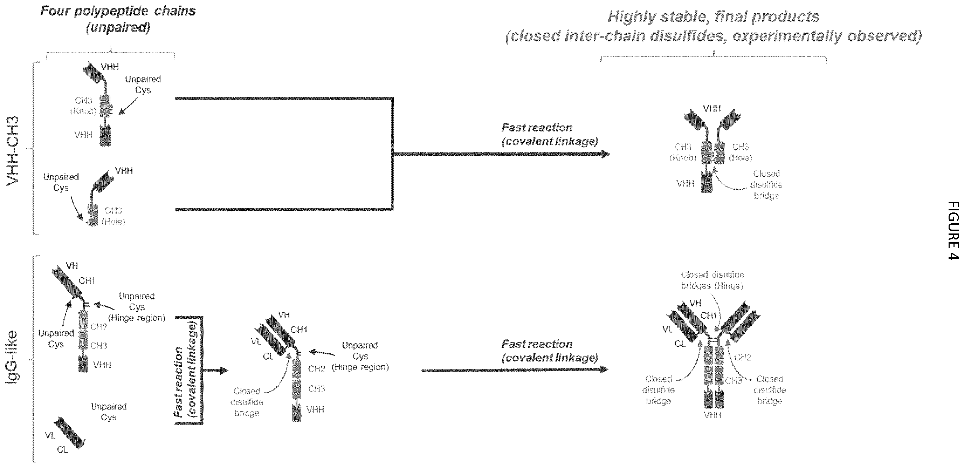

Lastly, the formation of inter-construct correct chain pairing was analysed (Figure 4).

CH3 domains exhibiting the Knobs-into-Holes mutations have a high affinity to one another and the formation is energetically favourable. In line with this, countless antibodies exhibiting Knobs-into-Holes mutations are investigated in clinical settings with even more antibodies exhibiting these mutations being in preclinical stages. Upon formation of the Knobs-into-Holes complex, an asymmetric disulphide bridge is formed and the fully functional VHH-CH3 fusion is generated. Due to covalent linkage, no dissociation event will occur.

Turning to the IgG-like construct, if the two HCs bind one another, the disulphides within the hinge region close and therefore lead to a covalently attached and stable IgG1-antibody.

In summary, the inventors consider that IgG-like molecules and VHH-CH3 fusions can be co-expressed using the invention with no occurrence of mispairing.

Given these theoretical findings, the inventors therefore proceeded to test the co-delivery system with these antibody constructs.

### Example 2 - Co-delivery of VHH-CH3 fusion and IgG-like antibody constructs

The present inventors elucidated for the first time the co-delivery of nucleotides encoding multiple antibodies and expression of only the correctly paired antibody chains.

The initial experiments investigated what type of mispairing of chains are observed if four plasmids encoding for the four polypeptide chains are co-transfected into ExpiHEK cells. Briefly, the plasmids encoding the VHH-CH3 fusion and the IgG-like antibody were transfected either alone or in all possible combinations into ExpiHEK293 cells. Supernatants were harvested and analyzed by Western Blot. The focus was on the detection of aggregates as well as on the formation of mispaired chains.

The results are summarised below.

Firstly, each single plasmid or each possible combination of two plasmids was transfected and the supernatant analysed for the presence of mispaired chains.

For the VHH-CH3 fusion, it was observed that the VHH-CH3(Knob)-VHH chains can be expressed solitarily and that no Knob-Knob homodimers are formed. VHH-CH3(Hole) chains cannot be produced solitarily. Co-transfection of plasmids encoding VHH-CH3(Knob)-VHH and VHH-CH3(Hole) chains resulted in the formation of the intended VHH-CH3 fusions, however, an excess of solitary free VHH-CH3(Knob)-VHH chain was observed.

For the IgG-like antibody, neither the LC nor the IgG(HC)-VHH were able to be produced solitarily. The co-transfection of plasmids encoding IgG(HC)-VHH and IgG(LC) led to the formation of the anticipated IgG1-like antibody.

Next, the inventors analysed the co-transfection of three of the final four plasmids in all possible combinations for the presence of mispaired chains.

When testing plasmids expressing both IgG(HC)-VHH and IgG(LC) in combination with a plasmid expressing either VHH-CH3(Knob)-VHH or VHH-CH3(Hole), it was observed that the presence of solitary Knob or Hole chains do not disrupt the formation of the IgG1-like antibody. When testing plasmids expressing both VHH-CH3(Knob)-VHH and VHH-CH3(Hole) in combination with a plasmid expressing either IgG(HC)-VHH or IgG(LC), it was observed that neither the presence of the IgG(HC)-VHH nor the presence of the IgG(LC) interferes with the formation of the VHH-CH3 fusion protein.

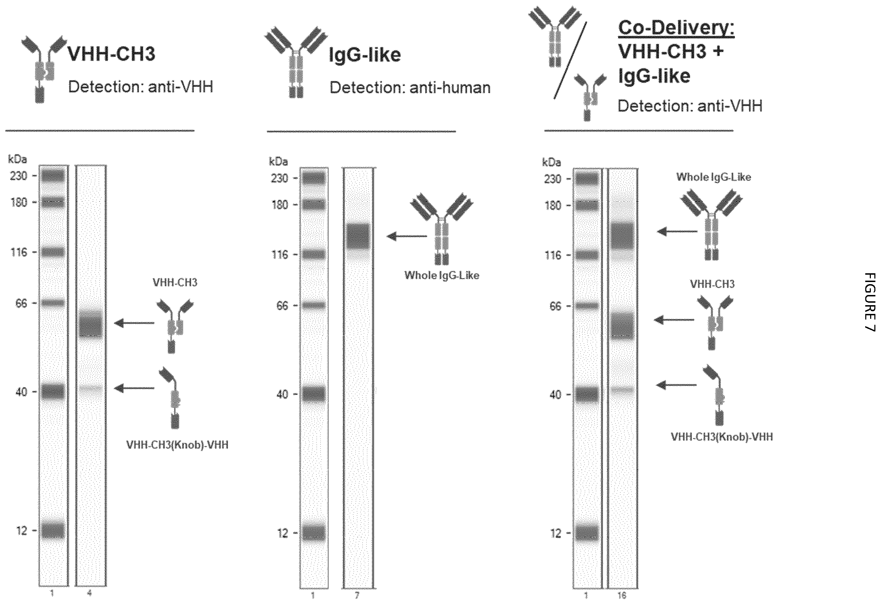

Lastly, the inventors tested the co-transfection of all four plasmids (see Figure 7).

As a control, the VHH-CH3 fusion and IgG-like antibodies were expressed separately as well as by co-transfection of all four plasmids.

The VHH-CH3 fusion is expressed very well on its own with no aggregates. Some free VHH-CH3(Knob)-VHH chain was observed.

For the separate expression of the IgG-like antibody, a clear band with no aggregates was observed, running at the expected size.

When all four plasmids were transfected together, VHH-CH3 fusions and the IgG-like antibody were successfully expressed. No aggregates were observed in the co-delivered constructs. No unexpected paired chains were observed. Co-transfected IgG-like antibodies and VHH-CH3 fusions look identical to the expression of the solitary expressed antibodies, i.e. their band patterns look like a mixture of both single construct transfections. However, also here the inventors observed the presence of the free solitary VHH-CH3(Knob)-VHH chain. Notwithstanding this, the experiment proved that the co-delivery of VHH-CH3 fusions and IgG-like antibodies is possible. The successful co-delivery facilitates a combination therapy with a single administration e.g. a single administration of a particle such as a LNP.

### Example 3 - Chain titration

The inventors have demonstrated that the co-delivery process is feasible, and no mispaired chains are observed. However, the VHH-CH3 fusion showed an excess of unpaired VHH-CH3(Knob)-VHH chain, which could be detected in the final co-transfected product as solitary polypeptide chain as a low molecular weight band. Furthermore, the inventors saw a higher expression level of the VHH-CH3 fusion compared to the IgG-like antibody. Whilst neither of these observations prevents the co-delivery of nucleotides expressing the two antibodies into a single cell followed by correct pairing of the antibody chains, the process could be improved by chain titration to eliminate the excess of VHH-CH3(Knob)-VHH chain and to balance the expression levels of the two antibodies.

Therefore, the inventors investigated titration of different molar ratios of all four plasmids and the supernatant of the cells were analysed by Western Blot.

All four plasmids of the VHH-CH3 fusion and the IgG-like antibody were co-transfected simultaneously at different ratios.

As the inventors saw before (see Example 2) that the VHH-CH3(Hole) chain is not expressed alone but the VHH-CH3(Knob)-VHH chain can be expressed and secreted without an interaction partner, the inventors strived to lower the concentration of the Knob chain compared to the Hole Chain.

As the heavy chain of an IgG can only be secreted with a light chain due to the interaction of CH1 with the chaperon BiP, whilst a light chain alone however can be secreted, the inventors aimed to reduce the concentration of the light chain compared to the heavy chain of the IgG-like antibody to avoid free or dimerized light chains.

To produce both antibodies in similar quantities, even though the expression level of the IgG-like antibody was higher in the initial experiments, a higher ratio of the VHH-CH3 fusion chains was tested.

The plasmids of the VHH-CH3 fusion and the IgG-like antibody were transfected at different ratios in ExpiHEK293 cells. Supernatants were harvested and analyzed by Western Blot. The focus was on the analysis of the presence of free chains and the relative expression level of IgG-like antibody and VHH-CH3 fusion. Two different VHH-CH3 fusions were tested, with each VHH-CH3 fusion comprising the same VHHs fused C-terminal to the CH3 domain but different VHHs (having specificity to the same antigen) fused N-terminal to the CH3 domain.

The results are summarised below.

The inventors chose one ratio for further testing that only contained the two desired bands:
0.8 VHH-CH3(Knob)-VHH : 2 VHH-CH3(Hole) : 1 IgG(HC)-VHH : 0.6 IgG(LC).

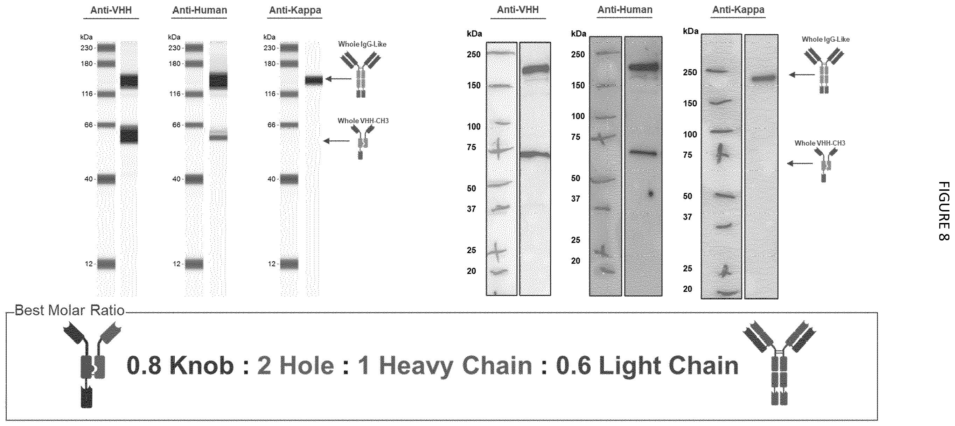

Only the two expected bands were observed for the IgG-like and the VHH-CH3 fusion with this ratio (Figure 8). No free knob chain was observed. No free light chain was observed.

By titrating the plasmid DNA ratios, the presence of free Knob chain was avoided.

In summary, it was identified that for this specific VHH-CH3 / IgG-like antibody pair an optimal molar plasmid ratio of 0.8 VHH-CH3(Knob)-VHH : 2 VHH-CH3(Hole) : 1 IgG(HC)-VHH : 0.6 IgG(LC) could be determined. This allows for the formation of only the anticipated molecules without free or solitary polypeptide chains in this particular case. This further proved that the co-delivery of VHH-CH3 fusions and IgG-like antibodies is possible and can be optimized using different molar ratios of plasmids. It has previously been shown that the optimisation of plasmid molar ratios is indicative of the RNA sequence ratios that can be used in RNA-based delivery systems encoding the same polypeptide chains. The titration data show that ratios of nucleic acid sequences can be optimized so that practically no unwanted side products are generated. These ratios are to be determined for each antibody combination individually.

### Example 4 - Validation of co-delivery of VHH-CH3 fusion and IgG-like antibody

In order to verify the functionality of the co-transfected antibodies, biolayer interferometry (BLI) measurements were performed next.

Both antibody constructs carry C-terminally VHHs targeting the same antigen (Antigen 1 (Ag1)). The supernatant was loaded onto SAX2.0 tips on which Antigen 1 tagged with biotin was previously immobilized. As binding of supernatant proteins to Antigen 1 was observed, the presence of Antigen 1-containing proteins in the supernatant could be confirmed.

The N-terminal VHHs of the VHH-CH3 fusions are specific to a second antigen (Antigen 2), so in the next step, soluble Antigen 2 was applied to the tip. As a binding event was observed only if the supernatant was applied before, the functionality of the VHH-CH3 fusion was verified.

To verify the functionality of the IgG-like antibody, instead of Antigen 2, CD64 was applied to the tip. CD64 binds to CH2 domains and can therefore only recognize the IgG1-like antibody and not the VHH-CH3 fusion, which lacks a CH2 domain. As again a binding event was observed, it was proven that both molecules are fully functional.

Two further assays were performed to analyze if inter-construct mispaired chains are present, but both turned out to be negative, further underlining that only the intended antibodies were produced.

### Example 5 - Co-transfection of two trispecific VHH-CH3 fusions and one bispecific IgG-like antibody

In this Example, the inventors look to verify that two different, multispecific VHH-CH3 fusions can be co-expressed with an IgG-like molecule without the formation of chain mispaired species. Furthermore, the functionality of all expressed molecules shall be confirmed.

In Examples 1-4, two antibodies with overall three specificities were produced (VHH-CH3: bispecific for Antigens 1 and 2; IgG-like: bispecific for Antigen 1 and Antigen 3).

Here, to further investigate the modularity of our system, the inventors strived to test the co-transfection of five plasmids. Again, the same VHH-CH3(Knob)-VHH chain as in the previous examples (i.e. specific for Antigens 1 and 2) was utilized, as well as the HC and LC-encoding plasmids of the same IgG-like antibody (i.e. specific for Antigens 1 and 3). Additionally, two plasmids either encoding for a VHH-CH3(Hole) chain comprising a VHH specific for Antigen 4 or for Antigen 5 were co-transfected. This would result in the formation of three different antibodies exhibiting five different specificities and being either bi- or trispecific (see Figure 5).

The plasmids of the two VHH-CH3 fusions and the IgG-like antibody were transfected at different ratios in ExpiHEK293 cells. Supernatants were harvested and analysed by Western Blot. The focus lay in the verification of the presence of free chains and the relative expression level of IgG-like antibody and VHH-CH3 fusions.

The first experiment comprised the testing of multiple molar plasmid ratios in order to investigate a suitable protocol to express all three antibodies of interest.

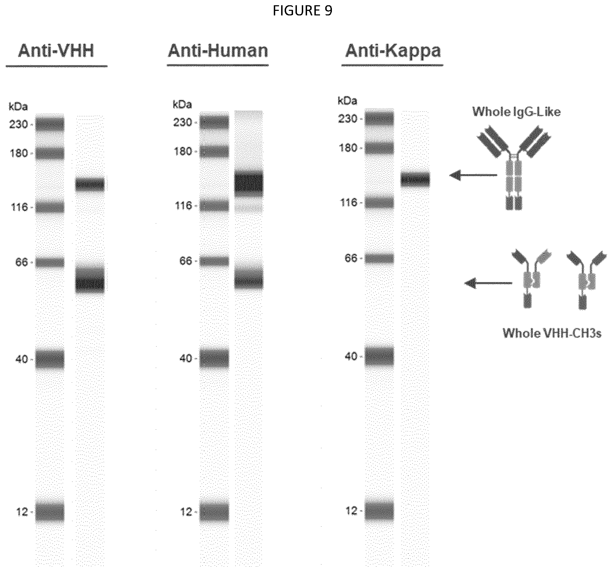

The results are shown in Figure 9 and summarised below.

Highly sensitive Jess Western Blots revealed the co-expression of two VHH-CH3 fusion constructs along with an IgG-like antibody, as expected. This validates that the co-expression of five different antibodies using the co-delivery system of the invention is achieved.

Depending on the ratio used, expression levels of the VHH-CH3 fusions and/or IgG-like antibody varies.

The best ratio was found at 1.2 VHH-CH3(Knob)-VHH : 1 VHH-CH3(Hole)1 : 1 VHH-CH3(Hole)2 : 1lgG(HC) : 0.4 IgG(LC). Only the two expected bands were seen for the IgG-like antibody and the VHH-CH3 fusions. No additional bands (i.e. no free knob chain and no free light chain) and no aggregation was observed.

The supernatant of the most promising ratio was tested in BLI to verify functionality of all expressed antibodies. BLI-assisted functional testing confirmed that both VHH-CH3 fusion proteins were fully capable of engaging all antigens when tested in the same supernatant. No mispaired bispecific VHH_CH3 Hole-Hole dimers were observed. By immobilizing the Fc part of the IgG-like antibody via anti-Fc antibodies and the subsequent binding to an Antigen 1-tagged protein, the presence and functionality of the IgG-like antibody was also confirmed.

### Discussion

Taken together, the inventors demonstrated a novel technology to co-express multiple antibodies within a single cell resulting in exclusive formation of the desired products.

FACS experiments showed that all of the tested co-expressed antibodies were able to bind to target positive cells. This underlines the full functionality of the co-delivery system developed by the present inventors.

By creating the novel VHH-CH3 fusions comprising KiH modifications and an asymmetric disulphide bond, the inventors fostered destabilization of mispaired chains and covalent stabilization of correctly paired antibodies. This allows for the formulation of multiple RNA sequences, encoding different antibody polypeptide chains, into a single LNP and the subsequent translation into fully functional and correctly paired multispecific antibodies.

In order for this system to work, differential stabilisations of the correctly paired antibodies are required. In the present Examples, the inventors employed in one antibody a heterodimeric Knobs-into-Holes CH3 pair, stabilized by an asymmetrical inter-CH3 disulphide bridge, in combination with an Fc portion of a second antibody, stabilized by hinge inter-chain disulphides. While the inventors utilized VHH-CH3 fusions and Fab-Fc-VHH antibodies in the present work, this technology also allows for the usage of other antibody formats utilising these same stabilisations of the correctly paired antibodies as well (Figure 6). Furthermore, alternative differential stabilisations of the correctly paired antibodies could also be employed, provided that stabilisations of mispaired chains are avoided.

The present inventors have also demonstrated that the N-termini of the antibodies can be fused to one or multiple different binding moieties like VHHs or Fabs. Alternatively, the N-termini can be left free.

Thus, this work provides a new co-delivery system which facilitates a combination therapy of multiple nucleotide encoded (e.g. mRNA encoded) antibodies expressed within a single cell resulting in exclusive formation of the desired products.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A co-delivery system comprising one or more nucleic acid molecules encoding a first antibody and a second antibody,
wherein each of the first antibody and the second antibody comprises at least a first polypeptide chain and a second polypeptide chain covalently linked by at least one inter-chain disulphide bond, and
wherein all of the inter-chain disulphide bonds between the first polypeptide chain and the second polypeptide chain of the first antibody are at different positions to the inter-chain disulphide bonds between the first polypeptide chain and the second polypeptide chain of the second antibody.

2. The co-delivery system according to claim 1, wherein:
a) all of the inter-chain disulphide bonds between the first polypeptide chain and the second polypeptide chain of the first antibody are within the hinge region; and/or
b) all of the inter-chain disulphide bonds between the first polypeptide chain and the second polypeptide chain of the second antibody are outside the hinge region.

3. The co-delivery system according to any one of the preceding claims, wherein:
a) the first polypeptide chain of the first antibody comprises at least one binding moiety or at least one cognate of a binding moiety at the N- and/or C-terminus;
b) the first polypeptide chain of the second antibody comprises at least one binding moiety or at least one cognate of a binding moiety at the N- and/or C-terminus;
c) the second polypeptide chain of the first antibody comprises at least one binding moiety or at least one cognate of a binding moiety at the N- and/or C-terminus; and/or
d) the second polypeptide chain of the second antibody comprises at least one binding moiety or at least one cognate of a binding moiety at the N- and/or C-terminus.

4. The co-delivery system according to any one of the preceding claims, wherein the one or more nucleic acid molecules further encodes one or more additional antibodies, preferably one, two, three, four or five additional antibodies.

5. The co-delivery system according to any one of the preceding claims, wherein the first antibody comprises an Fc region, preferably wherein the first antibody is an IgG antibody.

6. The co-delivery system according to any one of the preceding claims, wherein the first polypeptide chain and the second polypeptide chain of the second antibody each further comprises one or more constant heavy 3 (CH3) domains, preferably wherein the second antibody is a VHH-CH3 fusion.

7. The co-delivery system according to claim 6, wherein one or more of the CH3 domains of the first polypeptide chain and/or one or more of the CH3 domains of the second polypeptide chain of the second antibody comprise a modification that enhances the formation of the antibody, preferably wherein one or more of the CH3 domains of the first polypeptide chain and one or more of the CH3 domains of the second polypeptide chain of the second antibody are associated via a 'knobs-into-holes' modification.

8. The co-delivery system according to claim 6 or claim 7, wherein at least one inter-chain disulphide bond is formed between a CH3 domain of the first polypeptide chain and a CH3 domain of the second polypeptide chain of the second antibody, preferably wherein at least one inter-chain disulphide bond of the second antibody is an asymmetric disulphide bond.

9. The co-delivery system according to any one of claims 4 to 8 wherein each of the one or more additional antibodies comprises at least a first polypeptide chain and a second polypeptide chain, wherein the first polypeptide chain and the second polypeptide chain are covalently linked by at least one inter-chain disulphide bond, and wherein all of the inter-chain disulphide bonds between the first polypeptide chain and the second polypeptide chain of the one or more additional antibodies are at different positions to the inter-chain disulphide bonds between the first polypeptide chain and the second polypeptide chain in each of the first antibody and the second antibody.

10. The co-delivery system according to claim 9, wherein the first polypeptide chain and the second polypeptide chain of each of the one or more additional antibodies each further comprises one or more constant heavy 3 (CH3) domains, preferably wherein one or more of the CH3 domains of the first polypeptide chain and/or one or more of the CH3 domains of the second polypeptide chain of each of the one or more additional antibodies comprise a modification that enhances the formation of the antibody.

11. The co-delivery system according to claim 7 or claim 8, wherein each of the one or more additional antibodies comprises at least a first polypeptide chain and a second polypeptide chain, wherein the first polypeptide chain and the second polypeptide chain are covalently linked by at least one inter-chain disulphide bond, wherein the first polypeptide chain and the second polypeptide chain of each of the one or more additional antibodies each further comprises one or more constant heavy 3 (CH3) domains, wherein one or more of the CH3 domains of the first polypeptide chain and/or one or more of the CH3 domains of the second polypeptide chain of each of the one or more additional antibodies comprises a modification that enhances the formation of the antibody, and wherein the first polypeptide chain of the second antibody and the first polypeptide chain of each of the one or more additional antibodies is identical.

12. The co-delivery system according to claim 7 or claim 8, wherein the one or more nucleic acid molecules further encodes a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein all of the inter-chain disulphide bonds between the first polypeptide chain and the second polypeptide chain of the third antibody are at different positions to the inter-chain disulphide bonds between the first polypeptide chain and the second polypeptide chain in each of the first antibody and the second antibody, and wherein one or more of the CH3 domains of the first polypeptide and one or more of the CH3 domains of the second polypeptide of the third antibody comprise a modification that promotes electrostatic interactions between the first and second polypeptide chains of the third antibody.

13. The co-delivery system according to claim 7 or claim 8, wherein the one or more nucleic acid molecules further encodes a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond, wherein the first polypeptide chain and the second polypeptide chain of the third antibody each further comprises one or more constant heavy 3 (CH3) domains, wherein one or more of the CH3 domains of the first polypeptide chain and/or one or more of the CH3 domains of the second polypeptide chain of the third antibody comprises a modification that enhances the formation of the antibody, and wherein the first polypeptide chain of the second antibody and the first polypeptide chain of the third antibody is identical., preferably wherein the second polypeptide chain of the second antibody is different to the second polypeptide chain of the third antibody.

14. The co-delivery system according to any one of claims 1 to 8, wherein the one or more nucleic acid molecules further encodes a third antibody comprising at least a first polypeptide chain and a second polypeptide chain, wherein each of the first polypeptide chain and the second polypeptide chain of the third antibody comprises one or more constant heavy 3 (CH3) domains, wherein the first polypeptide chain and the second polypeptide chain of the third antibody are covalently linked by at least one inter-chain disulphide bond formed between a CH3 domain of the first polypeptide and a CH3 domain of the second polypeptide, and wherein all of the inter-chain disulphide bonds between the first polypeptide chain and the second polypeptide chain of the third antibody are outside the hinge region.

15. A vector comprising the co-delivery system according to any one of the preceding claims.

16. A nucleic acid particle comprising the co-delivery system according to any one of claims 1 to 14 or the vector according to claim 15.

17. A composition comprising the co-delivery system according to any one of claims 1 to 14, the vector according to claim 15 or the nucleic acid particle according to claim 16.

18. The co-delivery system according to any one of claims 1 to 14, the vector according to claim 15, the nucleic acid particle according to claim 16, or the composition according to claim 17 for use in a method of therapy and/or in a diagnostic method.
